# EUROPEAN PATENT SPECIFICATION

(11) **EP 0 937 140 B1**
(45) Date of publication and mention of the grant of the patent: **26.09.2007**
(21) Application number: 97931749.2
(22) Date of filing: 27.06.1997
(51) Int. Cl.: C12N 15/10, C12N 15/62, C07K 16/00, C07K 16/40, A61K 39/395, G01N 33/50

(54) **Antibody molecules which interact specifically with the active site or cleft of a target molecule**
Antikörpermoleküle, die spezifisch mit dem aktiven Zentrum oder dem aktiven Spalt eines Zielmoleküls interagieren
Anticorps ayant une interaction spécifique avec le site actif ou la fissure d'une molécule cible

(30) Priority: 27.06.1996 EP 96201788
(43) Date of publication of application: 25.08.1999
(73) Proprietor: Vlaams Interuniversitair Instituut voor Biotechnologie vzw., 9052 Zwijnaarde (BE)
(72) Inventor: MUYLDERMANS, Serge, B-1560 Hoeilaart (BE); WYNS, Lode, B-2000 Antwerpen (BE)
(74) Representative: Desaix, Anne
(86) International application number: PCT/EP1997/003488
(87) International publication number: WO 1997/049805

(56) References cited:
- WO-A-94/04678
- WO-A-96/34103
- DAVIES J ET AL: "ANTIBODY VH DOMAINS AS SMALL RECOGNITION UNITS" BIO/TECHNOLOGY, vol. 13, no. 5, May 1995, pages 475-479, XP002011336
- MUYLDERMANS S ET AL: "SEQUENCE AND STRUCTURE OF VH DOMAIN FROM NATURALLY OCCURRING CAMEL HEAVY CHAIN IMMUNOGLOBULINS LACKING LIGHT CHAINS" PROTEIN ENGINEERING, vol. 7, no. 9, September 1994, pages 1129-1135, XP002011333
- DAVIES J ET AL: "'CAMELISING' HUMAN ANTIBODY FRAGMENTS: NMR STUDIES ON VH DOMAINS" FEBS LETTERS, vol. 339, no. 3, 21 February 1994, pages 285-290, XP002011334
- HAMERS-CASTERMAN ET AL.: "Naturally occurring antibodies devoid of light chains" NATURE, vol. 363, 1993, pages 446-448, XP002048619 cited in the application

## Description

The present invention relates to recognition molecules, which are capable of interacting with the active site or cleft of target molecules. The invention further relates to methods for their design and preparation and use of the recognition molecules in diagnosis, therapy, vaccines and methods for isolation or purification of target molecules. Preferably the recognition molecule are used as enzyme inhibitors. The invention also relates to therapeutical compositions, diagnostic kits, vaccines and purification materials, comprising the recognition molecules of the invention.

In theory, in many instances the outbreak of diseases from viral, bacterial, parasitic or any other origin can be avoided by interfering with the enzymatic activity of pathogenic proteins or with the recognition of parasitic proteins with their target molecules. Furthermore, the deleterious effect of toxic substances can be counteracted by binding inhibiting molecules at the active (toxic) site. Also the malfunction of complex enzymatic or physiological processes finding their origin in a deregulated enzymatic function or deregulated protein recognition, often can be cured by adding molecules interacting with the active site or grooves of the complex proteins.

In all these examples it would be advantageous to have specific proteins recognizing the active site (such as the catalytic site in enzymes, grooves in proteins of complex systems, such as multicomponent systems, or recognition sites in receptors) of these malignant molecules.

Obviously the best technique at hand nowadays to obtain such molecules recognizing a particular target molecule is hybridoma technology for generating (monoclonal) antibodies. However, antibodies impose several limitations on their exploitation. It is for example known that antibodies of which the structure has been solved up to now, have an antigen binding surface forming either a groove or cavity itself or a flat surface (Webster et al., Current Opinion in Structural Biology, 4, 123, 1994). Thus, the antigen binding site of the antibodies cannot penetrate a groove or cavity. The catalytic or functional residues or toxic parts of the target proteins are located mostly within a cleft, so that the recognition of their substrate or receptor becomes very specific due to the many contacts and interactions with the amino acids forming the active cleft. However, due to the fact that clefts and cavities lie at least partially within the molecule, these structures are not very immunogenic. Even the wider cavities - or clefts of proteins in general - have the disadvantage that they are not very immunogenic. This is one of the main reasons why so few antibodies are interacting with the active site of proteins.

These (the low immunogenicity of the active site and the flat surface of the antigen binding site itself) are probably the two main reasons why so few (monoclonal) antibodies appear to have neutralizing enzymatic activity certainly when acting as monovalent fragments (F_{AB-}Fᵥ₋ScFᵥ), and this puts severe limitations on the great potential of monoclonal antibodies. The few neutralizing monoclonal antibodies that are available appear to bind on epitopes which overlap partially the active site of the antigen, but not inside the active site which would give them a greater specificity. Furthermore, a simple point mutation at the surface of the antigen (such as viral coat protein) removes the epitope of the monoclonal antibody which becomes useless for detecting this new variant. A last disadvantage of antibodies is their large molecular weight and size which impose limitations on the fast bio-distribution or efficient tissue penetration, while the Fc of antibodies prevents a fast clearance from blood.

In view of the above it is a first object of the present invention to design and construct molecules which bind with a great specificity to a cavity or active site of a target molecule.

It is a further object of the present invention to use these new molecules, which will be designated 'recognition molecules' throughout this specification, in diagnosis, therapy, vaccines.

The application also discloses methods for isolating or purifying target molecules as enzyme inhibitors and methods for preparing and modifying the recognition molecules for specific purposes.

The application discloses to therapeutical compositions, diagnostic kits, vaccines and purification materials, comprising the recognition molecules of the invention.

The first object of the invention is achieved by a recognition molecule as defined in the claims.

In such a recognition molecule the loop structure is the Complementary-Determining Region 3 (CDR3) of a camelid species heavy chain antibody having a binding specificity for the active site or cleft of the target molecule, or a modified version thereof.

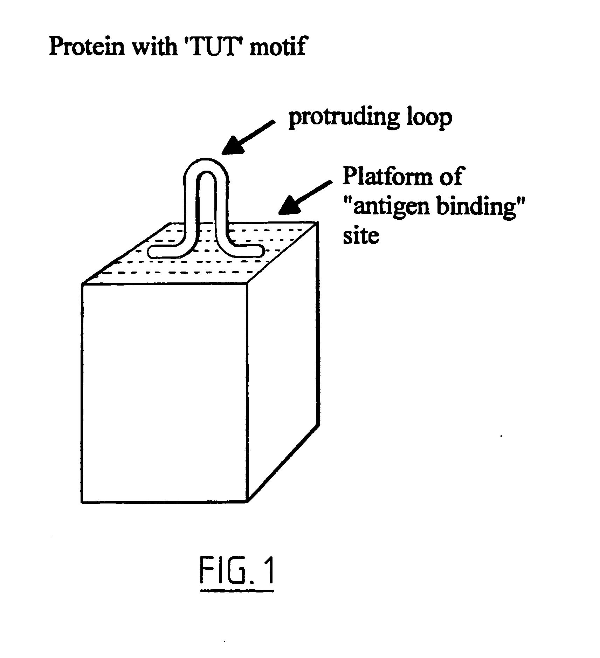

The invention was made after first having formulated the following principles. The recognition molecule of the invention should consist of an exposed loop protruding from a recognition unit (figure 1). The loop should be designed to penetrate inside a cavity, groove or cleft of the target protein. To have a good affinity this loop needs to be constrained so that its inherent flexibility is limited. Therefore the flexibility of the free loop needs to be restricted in absence of the target molecule. Although a restricted loop on itself might already have a sufficient affinity, it is an advantage (but not critically required) to have this loop protruding from a binding-surface to increase the number of contacts with the amino acids around the active site or cleft of the target protein. Thus, the ideal recognition molecule is something like the antigen-binding site of an antibody topped with an exposed loop protruding from this antigen-binding surface.

However, it was considered by the present inventors that the antigen binding site of conventional antibodies or antibody fragments such as Fv is not a good starting scaffold for insertion of an exposed loop because antibodies normally do not form loops protruding from their antigenic binding site and an artificially created loop is difficult to design de novo as the loop needs to be structured, constrained and should possess a complementary surface to the cavity of the target.

Moreover antibodies are too large for an efficient bio-distribution or tissue penetration, and antibody fragments such as Fv are rather unstable and easy to dissociate especially when used at lower concentrations.

Also, the much smaller VH antibody fragments or 'single domain antibody' (dAb) as they were called, (Ward et al., Nature 341, 544-546, 1989) derived from a conventional antibody have three major limitations, namely low expression yield in bacteria (only 0.2 mg/l culture on average), low solubility in aqueous solution, and reduced affinity and specificity compared to the parental Fv fragment.

The molecule of the present invention should penetrate into the active site of the target protein, where it interacts with the catalytic residues, although other cavities, grooves or clefts of the target protein will do as well. To this end the molecule of the invention should possess an exposed loop, large enough to insert maximally, and as complementary as possible to the target protein cavity. The good fit, and high number of contacts of the recognition molecule with the active site residues of the target molecule should make it impossible for the latter to escape this interaction by the acquisition of point mutations as these will have deleterious effect on the proper function of the target molecule itself. The recognition molecule of the invention is further to be characterized by a small size for good bio-distribution and tissue penetration, sufficient high expression level in bacterial systems for economical reasons, a good solubility behaviour, a good stability and a long shelf-life time, a good affinity and specificity for the target molecule, an easy cloning and downstream manipulation.

The present inventors surprisingly found that all these requirements can be met when starting out from the heavy chain antibodies from camelids (Camel bactrianus, Camel dromedarius, Lama peruviana, Lama glama, Lama vicugna and Lama alpaca). Camelids contain a substantial amount of their functional antibodies in the form of heavy-chain antibodies only (Hamers-Casterman et al., Nature 363, 446, 1993). The heavy-chain antibody is composed of homodimers of H chains and lack L chains. From the amino acid sequence of the H chains it appears that their N-terminal domain harbours some remarkable amino acid substitutions which make them clearly distinct from the conventional VH (Muyldermans et al., Prot. Enging. 7, 1129, 1994).

To make a clear distinction between the conventional VH and that of camelids, the heavy-chain antibody of the latter is identified as 'VHH' (VH of Heavy-chain antibody). This distinction is entirely justified as follows from the fact that camelid heavy-chain antibodies are clearly different from any other VH.

The fact that the camelid germline contains both a V_{H} and a V_{HH} set of minigenes prves that the V_{HH} domains (obtained after V_{HH}-D-J-recombination) are predestinated for usage in heavy-chain antibodies devoid of light chains.

Although the amino acid substitutions in a VHH compared to any other VH are scattered throughout the primary structure (sequence) they are clustered in space in the tertiary structure at the side of the molecule which normally interacts with the VL domain (referred to as 'former VL side'). These amino acid substitutions are V37F, G44E, L45R or L45C and W47 mostly substituted in Gly. Evidently, such substitutions are expected to render the 'former VL side' of the VHH more hydrophilic and therefore they will overcome the solubility limitations of the conventional isolated VH's obtained from human or mouse also referred to as dAb (single domain antibodies). Ward et al., Nature 341, 544 (1989) described the so-called dAb's.

The substitutions also make that this region is less likely to bind to the chaperon BiP (or bacterial chaperon proteins) so that it is expected that the expression level might also increase.

Moreover, as the camelid heavy chain antibodies were matured in vivo in absence of any light chain, it was anticipated that the isolated VHH will retain the parental affinity and specificity for its antigen of the original heavy chain antibody.

In conclusion, VHH's have at least three main advantages compared to the isolated VH's or dAb's, namely better expression yield in bacteria or other expression systems, a better solubility in aqueous solutions and an increased affinity and specificity.

Indeed, it was demonstrated in the research that led to the present invention that a VHH when cloned in a bacterial expression vector (pHEN4, a derivative of the pHEN1 (described by Hoogenboom et al., Nucl. Acids Res. 19, 4133 (1991))) can be expressed to yield approximately 10 mg/l culture. This should be compared to 0.2 mgr/l culture for bacterial expression of isolated mouse VH domains. The cause of these unfavorable properties of mouse dAb's is the exposure to aqueous solvent of the hydrophobic face of 'former VL side'. The VHH could also easily be concentrated to 10 mg/ml without any sign of aggregation, which corresponds to an approximately a 100 times higher solubility than that of mouse VH's.

Besides the good bacterial expression and solubility it was further shown that the VHH was resistant against thermal denaturation and could be kept at 37°C for up to 1 week with retention of structural integrity and antigen binding capacity. It was therefore concluded that the VHH are stable molecules.

It is possible to camelise ordinary VH's to equip them with the advantages of VHH. The so-called 'camelisation' involves the mutagenesis of amino acids at position 44, 45 and 47 so as to mimic the corresponding camel amino acids at those positions. 'Camelisation' improves on the solubility (Davies & Reichmann FEBS Lett. 339, 285-290, 1994). Further 'camelisation' of position 37 by V37F substitution and the introduction of a disulphide bond between the CDR1 and CDR3 improved considerably the stability of the isolated domain.

The sequence analysis of additional VHH clones (from camel and llama) revealed some remarkable additional features about their functionality, especially how they retained a specific antigen binding capacity in the absence of any light chain antigen binding loops. In a conventional VH, it is the CDR1 (amino acid 31 to 35) which is hypervariable in sequence and found to contact the antigen. The N-end in front of the first hypervariable loop (amino acid 26 to 30) is solvent exposed in a conventional VH, however, these amino acids are conserved and were never before reported to contact the antigen (with the exception of amino acid at position 30).

In the VHH of camelids these amino acids at position 26 to 35 can be defined as hypervariable in sequence. This suggests, first, that the amino acids of this loop can adopt a different conformation than that described up to now in other species, and second, it indicates that those amino acids might contact the antigen, i.e. the surface of the antigen binding platform would be enlarged.

Furthermore, it was found that the CDR3 which is the most variable loop in sequence and in structure is on average longer than that of conventional VH domains (15 amino acids compared to 9 amino acids in mice). Again an increase in the antigen binding surface is anticipated.

A drawback of a longer loop in absence of the antigen means that the loop has some flexibility and may adopt more different conformations of which one becomes fixed upon complexation with the antigen. This immobilisation of the loop will have a large negative entropic effect on binding. The frequent occurrence (especially in the longer loops) of cysteines simultaneously in the CDR1 (or CDR2 or position 45 of framework 2) and the CDR3 of camelid VHHs is in accordance with the formation of a disulphide bond. This will reduce the conformational flexibility and therefore the antigen binding will have less negative entropic contribution in binding.

From the results of VHH strategiy can be proposed for generating functional small recognition units.

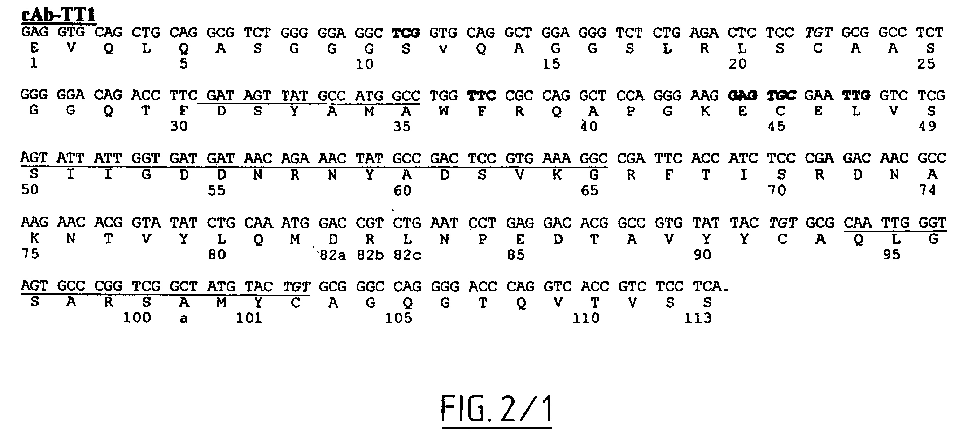

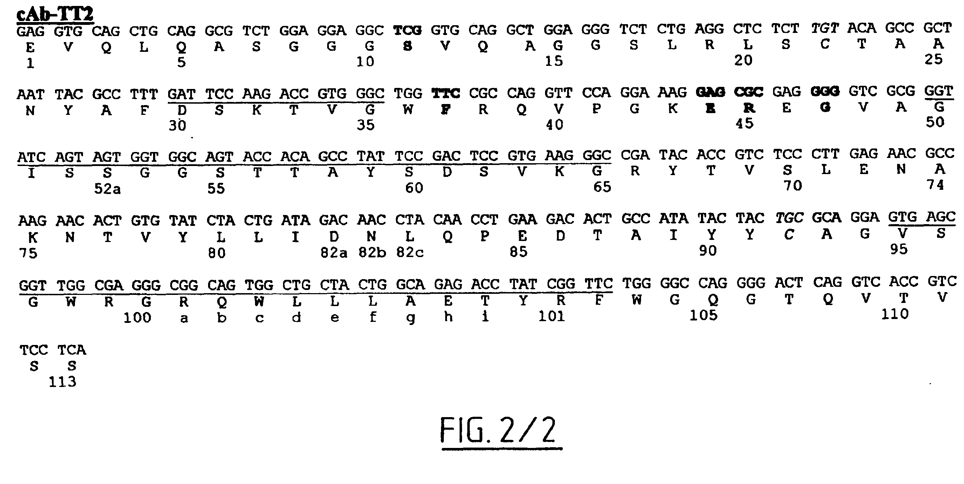

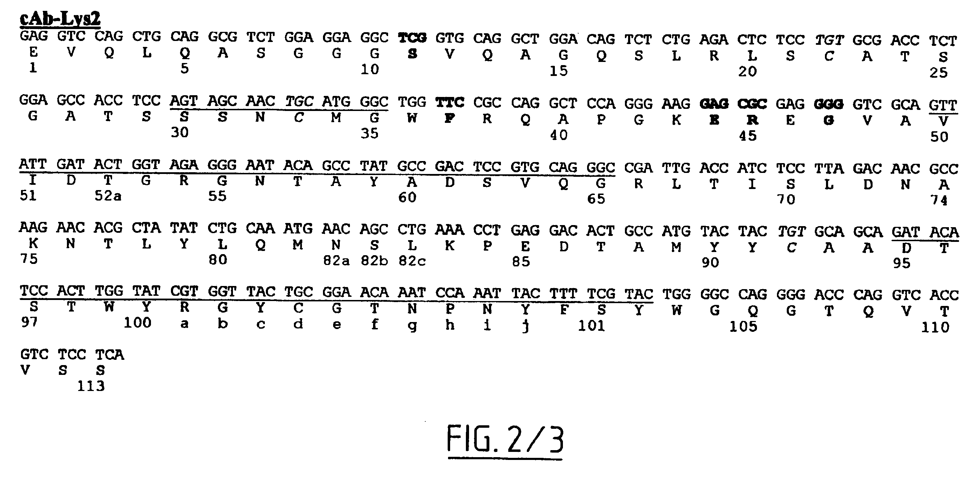

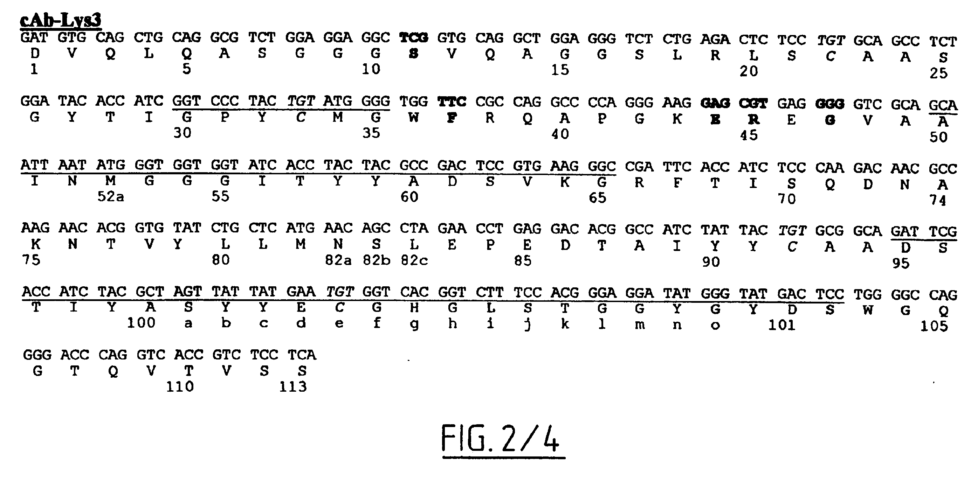

The strategy comprises immunising a camel (or llama) with the desired antigen so that the immune system of the animal will mature his heavy-chain antibodies in vivo. Subsequently the VHH from the lymphocytes (blood, spleen, bone marrow) are cloned in a phage display vector such as the pHEN4, and selected by panning with the antigen. Using this technique successful identification of two VHH molecules binding to different epitopes of lysozyme and two VHH binders binding to tetanus toxoid, one to the C-fragment and the other outside the C-fragment was possible. These VHH were called cAb-Lys2, cAb-Lys3 and cAB-TT1 and cAb-TT2, respectively. See figure 2 for their amino acid and nucleotide sequences with Kabat numbering. (cAb stands for camel single domain antibody fragment.)

The cAb's are specific for their antigen, and bind to it with affinities of 2.10⁸, 2.10⁷, 6.10⁷ and 2.10⁷ M⁻¹ respectively. The bacterial expression levels of these cAb's are always in the mg/l culture range, the cAb's are well folded and behave quite soluble and stable in thermal denaturation experiments.

It is possible to increase their affinity by making the cAb's bivalent/multivalent by the intermediate of the camel long hinge. In a similar strategy the cAb-Lys3 can be linked to the cAb-TT2 to generate bispecific constructs. The cAb-TT1 and cAb-TT2 are shown to neutralise the tetanus toxin in vivo. The cAb-Lys3 is inhibiting the Micrococcus Lysomeikticus cell wall hydrolysing activity of lysozyme as well.

The cAb-Lys3 is readily purified by affinity chromatography on hen egg-white lysozyme immobilised on Sepharose CNBr. The structure of the cAb-Lys3 in complex with its antigen (hen egg-white lysozyme) was determined to 2.5 Å resolution by X-ray crystallography. The main observations of the cAb-Lys3 structure with respect to the development or design of VHH as small recognition units with the exposed loop structure (also called 'TUT' motif) are the following.

The main chain conformation of the core of the VHH is similar to the VH, so that it can be envisaged to use the VHH for grafting the CDR's from other useful conventional VH molecules.

The 'former VL' side of the cAb-Lys3 is completely reshaped and became more hydrophilic compared to the VH, due to the substitution of the V37F, G44E, L45R and W47 mostly substituted in Gly, but also due to the reorientation of the conserved W103, Q105 and Q39 in this region.

The H1 loop has a conformation which deviates completely from the described canonical structure 1 of conventional VH's, and amino acid 29 which in conventional VH domains is buried in the interior of the domain flips out the structure and contacts the antigen. Also the amino acid 28 is close to the antigen in the complex.

The CDR3 (24 amino acids long in cAb-Lys3) can be divided into two parts, the C-part covering the 'former VL side', and the N-part forming an exposed, accessible, extended and protruding loop (referred to as the 'TUT'). This loop is stabilised by a disulphide bond (towards the CDR1) and an internal aromatic core formed by a clustering of Y32, Y99 and Y100c. The Y99 is also making a H-bond with the side chain of D95.

The loop at amino acids 72-75 is close to the antigen, but is not very well ordered.

The exposed part of the CDR3 loop penetrates deeply inside the active site of the lysozyme, and the tip of the loop formed by Ala100 and Ser100a. The Ser100a makes a H-bond with the catalytic Glu35 of lysozyme.

It was found that the stabilised and large protruding loop (the 'TUT') interacts with the active cleft of the lysozyme, an enzyme region considered to be a 'low energetic epitope', because of which it is difficult to raise antibodies against this part of the molecule. The active site of the enzyme, or cavities of protein surface in general, are difficult to react with conventional antibodies or antibody fragments due to their low immunogenicity or because the antigen binding site is either flat or carries a cavity or groove, but a large protruding loop was not observed on the antigen binding site of antibodies so that they cannot penetrate cavities, clefts or grooves of the antigen.

A broad range of proteins or other molecules can function as target molecules. A list of proteins is given merely as an indication of which kind of proteins can be selected. All other proteins with a cavity or small groove are as good, and the list is certainly not limited.

Examples of target molecules are bacterial toxins, such as Toxic Shock Syndrome Toxin 1 of S.aureus, which is a member of a large family of toxins secreted by S.aureus and is the major cause of toxic shock syndrome. TSST-1 has 20-30% sequence identity with staphylococcal enterotoxin B proteins, cholera toxin, tetanus toxin. Other molecules that may be selected as target molecules are snake venoms, such as adamalysin II, a smaller protein, which is a zinc endopeptidase from rattlesnake and consists of a highly conserved catalytic domain, or Cardiotoxin CTX IIb (Naja mosambica), Cardiotoxin CTX V (Taiwan Cobra). These cardiotoxins are small proteins in the venoms of snakes from the Elapidae family. The toxins are known to bind and disrupt the organization, integrity and function of the cell membrane. Others are Dendrotoxin K (Black mamba), Flavoridin Neurotoxin-I and II (Asian cobra). There is also a sequence similarity of adamalysin II to the low molecular weight metalloproteinase Ht-c and Ht-d from the Crotalus atrox which degrades type IV collagen.

Other target molecules are for example receptors. Receptors are biological macromolecules capable to bind a complementary biomolecule, or counterligand, resulting in a function (through, e.g., signal transduction or storage and subsequent release). The recognition molecules of the invention can be used as antagonists to receptors in order to block for example the signal transducing function thereof. As an alternative the recognition molecules can have a agonistic activity.

Target molecules can furthermore be honey bee venoms, such as apamin and tertiapin, spider toxins, viral and bacterial specific proteins, such as HIV protease, HIV reverse transcriptase, SIV protease, alkaline protease from Pseudomonas aeruginosa, other proteases, such as serine proteases like Factor Xa or other blood serine proteases, RNases and angiogenin, sialidases thought to be involved in the pathogenesis of many diseases (Salmonella, influenza virus), which catalyse the cleavage of glycosidic linkages between sialic acid and glycoconjugates, amylases and β-glucanases, which catalyse the hydrolysis of glycosidic linkages of various oligosaccharides. Changes in α-amylase activity are often indicative of pancreatic disorders. The active site of the enzyme forms a cleft that lies between the A and B domains. The catalytic residues Asp300, Asp197 and Glu233 are located at the cleft and homologous residues have been found within several amylase structures.

Other examples of target molecules are lysozyme, tetanus toxin and carbonic anhydrase.

Starting from a basic recognition molecule as defined in the claims consisting of a basic recognition unit and a loop, structure variations can be made to make recognition molecules for other targets. Furthermore, a selection system can be designed to select suitable candidates for a desired target from within a large group of variants.

In principle every recognition molecule can be used as a starting point for this approach. However, to avoid further immunization use can be made from one of the recognition molecules described herein. Such a molecule can then be engineered to obtain the desired specificity.

Variations leading to modified versions of the loop or the basic recognition unit can be made in various ways. For example, the derived version of the CDR3 may be a mutated CDR3 in which at least one of its native amino acids is replaced by one or more other amino acids. Or as an alternative the derived version of the CDR3 consists of a mutated CDR3 in which one or more additional amino acids are added to and/or incorporated within its native amino acid sequence.

Similar modifications can be made to the basic recognition unit. For example, when the basic recognition unit is an antibody-type structure formed by at least part of a camelid species heavy chain antibody, the application discloses a modified version thereof that is a version in which at least one of its native amino acids is replaced by one or more other amino acids, or a version in which one or more additional amino acids are added to and/or incorporated within its native amino acid sequence. As an alternative the modified version of the camelid species may comprise a version which is fused to a second amino acid sequence or a biologically active molecule.

In order to obtain recognition molecules of the invention various strategies can be followed. In general a first method comprises providing a camelid heavy chain antibody; isolating and cloning the coding sequence therefore in a phage display vector; expressing the coding sequence on a phage harbouring the vector; and selecting the recognition molecule specific for the antigen by panning the phage with the immobilized antigen.

The first strategy thus consists of immunizing a camel (or llama) with the desired target antigen so that the immune system of the animal matures his heavy-chain antibodies in vivo against this immunogen. Subsequently, the VHH's from the lymphocytes (blood, spleen, bone marrow) are cloned in a phage display vector such as the pHEN4, and selected by panning with the immobilized antigen. To elute the binders (recognition molecules that bind the desired antigen) one of the following methods was chosen.

The elution of binders is performed by a pH shock to obtain general binders (not only active site binders). The elution of binders is performed with an excess of substrate if one only wishes to obtain the cavity or active site binders. By bringing the 'general binders' from the first method over an immobilized target protein/substrate complex and continuing with the non-binders the cavity binders are selected from the general binders.

The feasibility of this general method to obtain recognition molecules of the invention was proved by obtaining the camel single-domain antibody (cAb) fragments cAb-TT1, cAb-TT2, cAb-Lys2 and cAb-Lys3 proteins using this strategy. The structure analysis of the cAb-Lys3, which has the longest CDR3 loop (24 amino acids) of all lysozyme binders and a cysteine forming a disulfide bond between the CDR1 and CDR3, proved that indeed a small recognition unit with a protruding loop binding into the active site of the enzyme was generated as anticipated.

It is possible to repeat the first strategy for obtaining recognition molecules with other 'target' proteins but not all proteins are sufficiently immunogenic, to completely generalize this strategy. Therefore, a second strategy was developed.

This strategy uses a randomly chosen recognition molecule as a starting point. The advantage thereof is that immunization can be avoided, and the tertiary structure of the final molecule is already essentially provided for.

Therefore, such a second method comprises in general selecting a random camelid heavy chain antibody; isolating and cloning the coding sequence therefore in a phage display vector; modifying the coding sequence of the exposed loop by random substitution of at least one of the codons thereof; preparing a library of randomly mutated coding sequences in phage display vectors; expressing the coding sequence on phages harbouring the vector; and selecting the recognition molecule specific for the antigen by panning the phage with the immobilized antigen.

For this second strategy, the camel immunization is avoided. For example, the cAb-Lys3 protein is modified to develop 'small recognition units' with a 'TUT' motif for binding into the target protein clefts. Two routes are envisaged: a first one in which the protruding loop is reshaped and secondly a route ending in a 'veneering' of the cAb-Lys3 protruding loop.

To reshape the loop several steps need to be undertaken. First, introduction of restriction enzyme sites in the vicinity of the loop. These sites help in the subsequent cloning and characterization. Then, exchange of the amino acids of the loop by a random codons (1 to X). The smaller the number X, the smaller the library, and the shorter the extension of the loop. Loops of more than 6-7 amino acids might be more difficult to generate a complete library due to the experimental limitations in bacterial transformation efficiency, and also the loop might become too flexible which will result in polyreactivity and make less tight binders. Subsequently, exchange the platform around the loop by changing the N terminal end of the domain, the CDR1, the CDR2 or the loop around amino acids at position 72/75 (Figure 2, cAb-Lys3) in order to increase the specificity or affinity. The previous steps 2 and 3 can be repeated cyclically for affinity and specificity maturation. Alternatively multivalent constructs can be made as well to increase the avidity or to obtain bispecific constructs.

For 'veneering' the cAb-Lys3 CDR3 loop the following steps are performed. First, introduction of restriction enzymes sites around the loop for cloning or characterization purposes. Then, substitution of the amino acids which are exposed on the outside of the protruding loop of the cAb-Lys3. Finally, exchange of the platform around the loop by changing the N terminal end, the CDR1, the CDR2 or the amino acids around the 72/75 loop in order to increase the specificity or affinity. Steps 2 and 3 might be repeated for affinity and specificity maturation. Alternatively multivalent constructs can be made as well to increase the avidity or to obtain bispecific constructs.

It is also possible to produce the recognition molecules of the invention by means of standard genetic engineering techniques, such as expression of a DNA sequence encoding the recognition molecule. Such a method may for example comprise the steps of isolating a DNA sequence encoding the recognition molecule or a precursor therefor; optionally modifying the molecule or the precursor by introducing one or more base substitutions, deletions or insertions; transferring the thus obtained optionally modified DNA sequence to a suitable host; and expressing the DNA sequence in the host. The term 'precursor' as used herein intends to encompass every sequence that does not have the (complete) desired specificity of the recognition molecule to be produced.

It is evident that with these approaches the problems of immunization (long immunization schemes, toxic effects of immunogens to camelids, low immunogenicity of the target molecule, need for relatively large amounts of target molecules for immunization) are avoided.

According to another aspect thereof the invention relates to use of the recognition molecules in neutralising the biological function of the target molecule, and in therapy. The invention thus also relates to a therapeutical composition, comprising one or more recognition molecules of the invention and a suitable excipient.

Apart from neutralising, the recognition molecules may also be used to detect the presence of the target molecule in a sample. As such the recognition molecules may be used for diagnosis. The recognition molecules can be used analogous to conventional antibodies. Similar diagnostic techniques are therefore envisaged here, without further explanation, because the skilled person will be very well capable of designing every conceivable diagnostic test in analogy to known immunological diagnostic tests. The invention thus can be used for providing diagnostic test kits, comprising one or more recognition molecules.

The recognition molecules, like conventional antibodies may find application in (passive) vaccines. To this end, the invention relates to the use of one or more recognition molecules for immunization.

Furthermore, the specificity of the recognition molecule for the target molecule can be deployed to isolate or further purify the target molecule. Use can be made of standard separation and purification techniques, such as affinity columns, wherein the conventional antibody or other binding molecule is substituted with the recognition molecule of the invention. The application discloser further purification material, consisting of a carrier having one or more recognition molecules of the invention bound thereto. Preferably, the carrier is column material preferably an affinity column.

The application discloses that once the small recognition unit with the 'TUT' motif (recognition molecule of the invention) is constructed, it can thus be used immediately in a number of applications, for example instead of a conventional monoclonal where a rapid clearance from blood of excess molecule is advantageous. However, for other applications it might be preferable to increase the lifetime in circulating blood. This is readily obtained by cloning the recognition unit in front of the hinge, CH2 and CH3 domains of human IgG1.

The application discloses that in a third class of applications it might be necessary to turn these small recognition units into intrabodies. The small size, and their single domain architecture makes that they are suitable for such use.

Cloning of a SKDEL motif at the end of the gene segment will keep the molecule inside the endoplasmic reticulum, or cloning behind a nuclear target signal, the chloroplast signal, will bring the protein inside the nucleus, the chloroplast, the mitochondria or any other selected organelle where it should bind and inactivate its target.

For a number of cases, the binding activity of the loop on itself might be sufficient for specific interaction inside the active cleft of the target molecule.

The loop can also be used to rationally design peptido-mimics preferably mimicking the properties of natural proteins.

The present invention will be further elucidated referring to the following figures and

### examples.

### DESCRIPTION OF THE FIGURES

Figure 1 shows a schematic representation of a recognition molecule of the invention.
Figure 2 shows the amino acid and nucleotide sequences of cAb-Lys2, cAb-Lys3, cAb-TT1 and cAb-TT2.

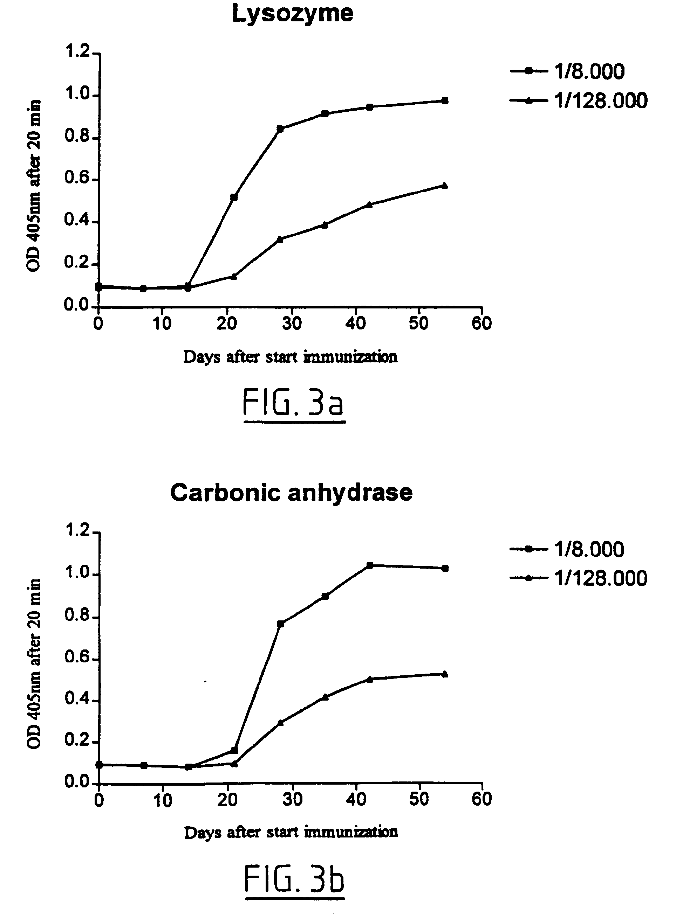

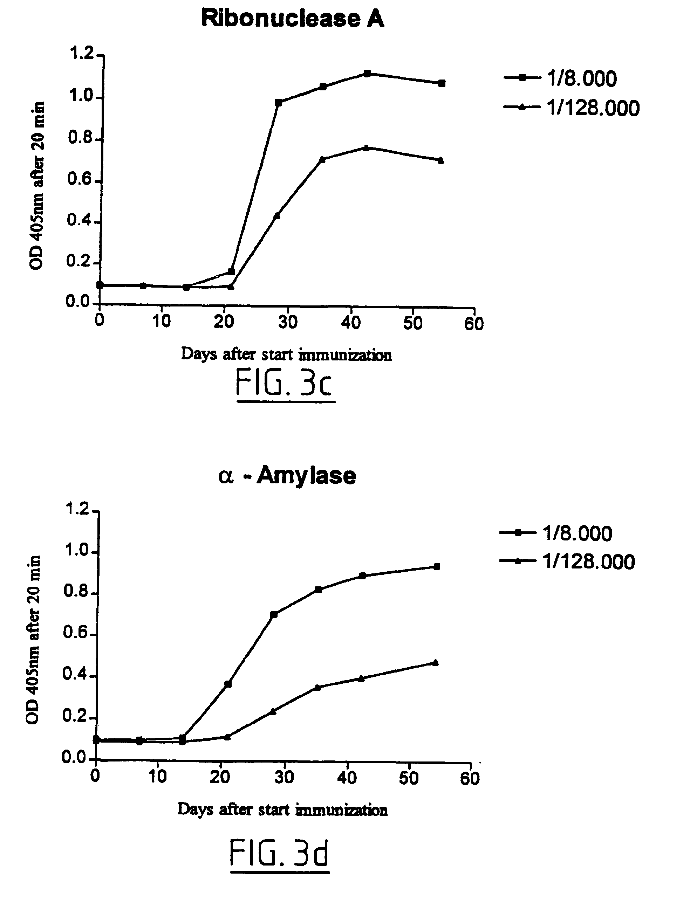

Figures 3A, 3B, 3C and 3D shown the immune response in functional time for lysozyme carbonic anhydrase bovine erythrocytes α-Amylase. See Example 12.
Figures 4A, 4B, 4C and 4D show the solid-phase binding of fractionated IgG of D2/54 for RNaseA of the Amylase lysozyme and carbonic anhydrase. See Example 13.

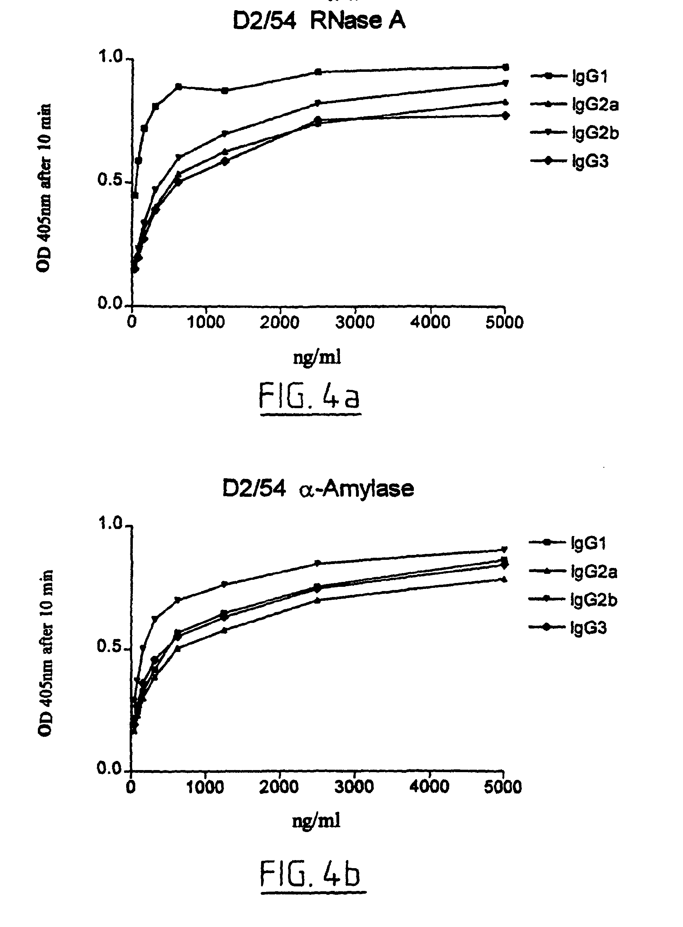

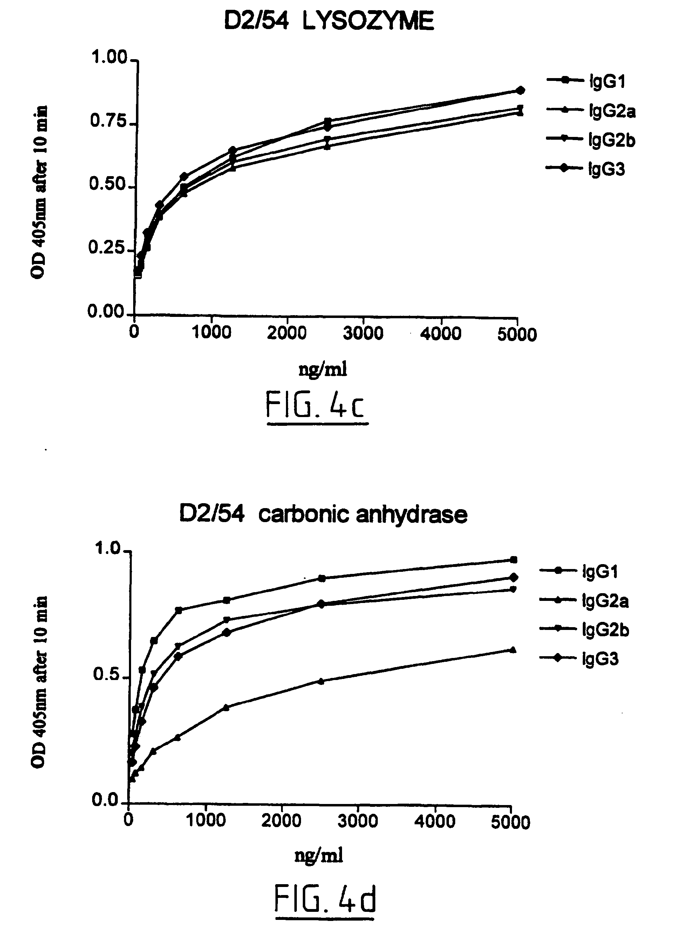

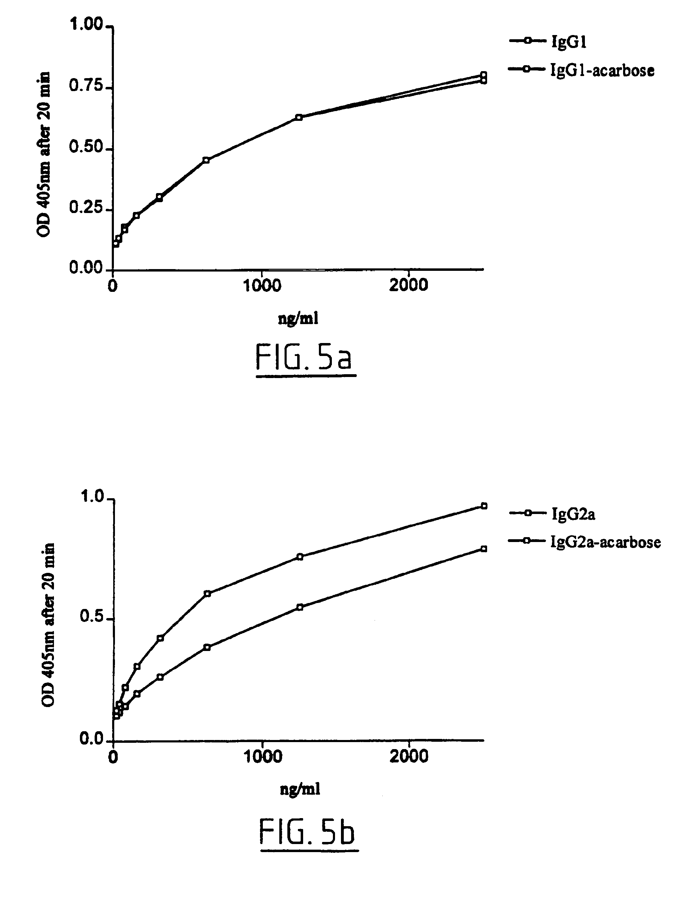

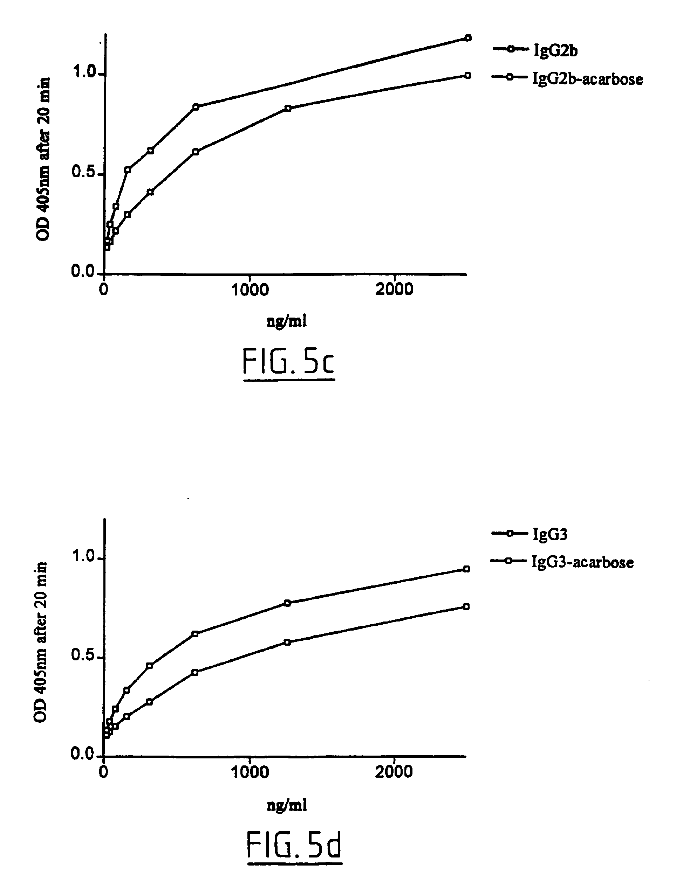

Figures 5A, 5B, 5C and 5D show the optical densities of bovine and pancreatic α-Amylase for differing IgG's. See Example 14.

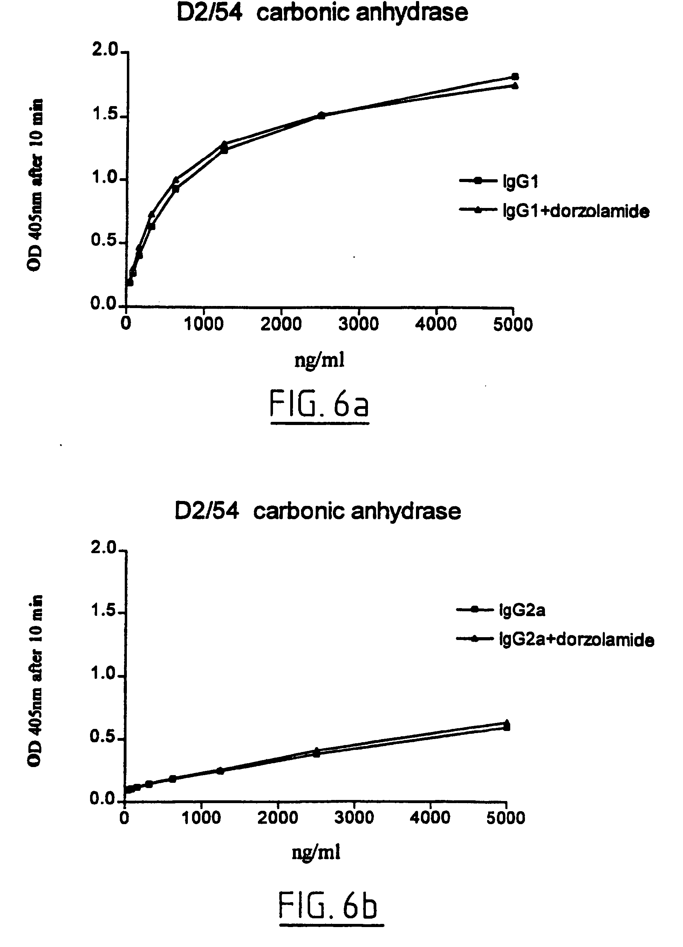

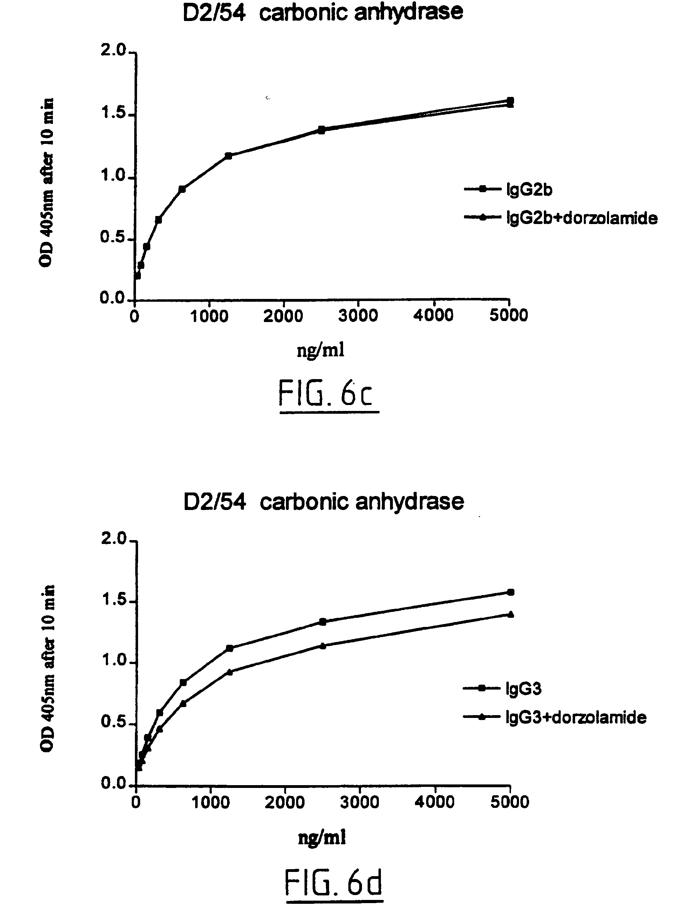

Figures 6A, 6B, 6C and 6D show optical densities for bovine erythrocyte carbonic anhydrase for differing IgG's. Also see Example 14.
Figure 7 shows the chromatograph obtained in Example 16.
Figure 8 shows a gene sequence. See Example 18.

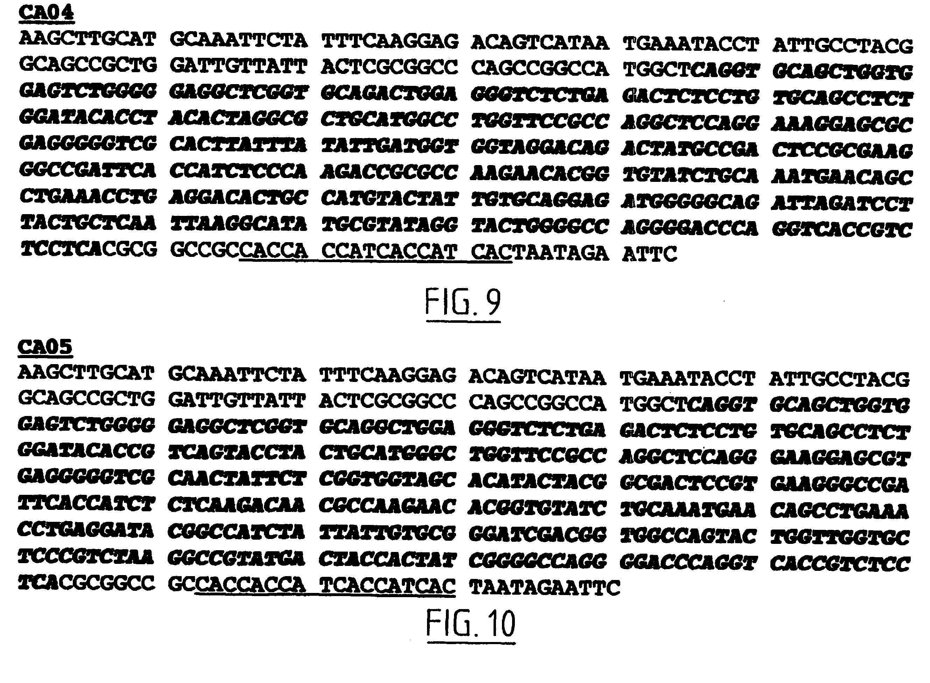

Figures 9 and 10 show the gene sequences for respectably CA04 and CA05. See Example 19.

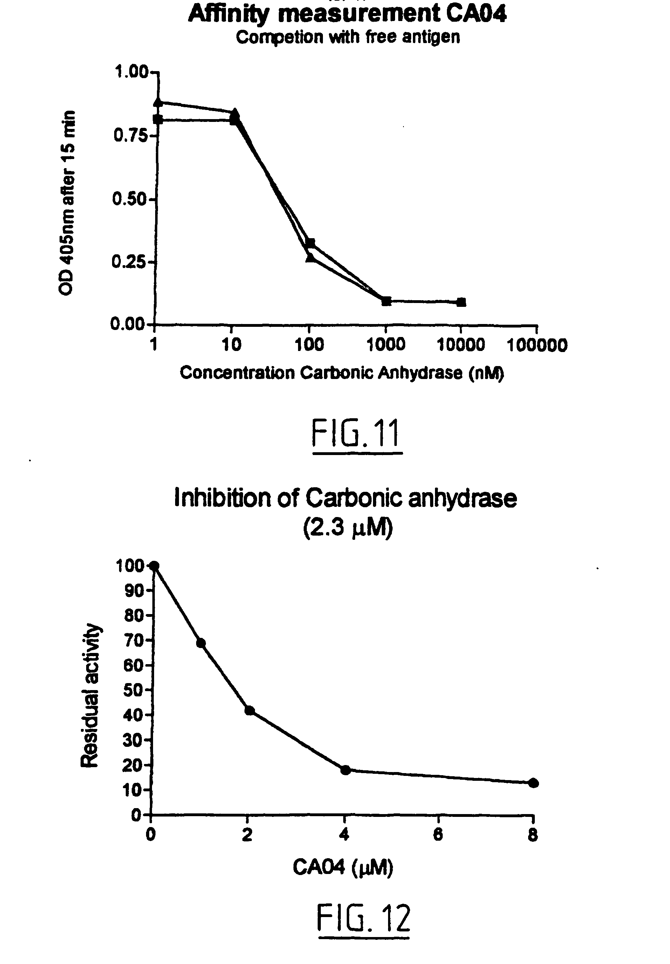

Figure 11 shows a graph showing the affinity measurement of CA04-HIS construct by competitive ELISA. See Example 20.
Figure 12 shows a graph exhibiting the inhibition of carbonic anhydrase. See Example 22.

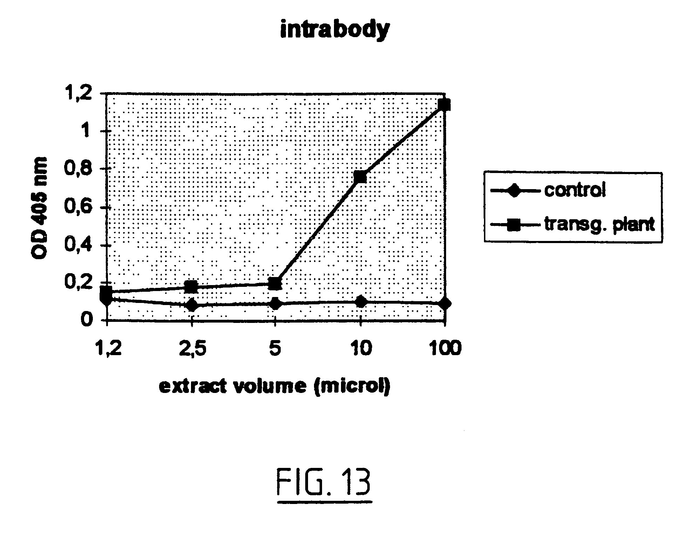

Figure 13 is a graph as explained in Example 7.

### EXAMPLES

### EXAMPLE 1

### Preparation of cAb-Lys2 and cAb-Lys3

The procedure to obtain cAb-Lys2 and cAb-lys3 is disclosed in the patent application WO 96 34103 published on October 31, 1996.

### EXAMPLE 2

### Introduction of restriction enzyme sites in the vicinity of the N-part of the CDR3 loop of cAb-Lys3

The pHEN4-αLys3 (i.e. the plasmid of pHEN4 containing the gene for camel VHH coding for the cAb-Lys3 protein) was taken as a template and a PCR was performed with the VHBACK(A4) and the SM020 primers. Another PCR is performed on the same template DNA and with primers SM019 and AM006.
AM006 binds in the beginning of the gene pIII of pHEN4:
5'-CGTTAGTAAATGAATTTTCTGTATGAGG-3'
SM019 binds to codons 100g to 100m of cAb-Lys3 (Sal I site underlined):
5'-CACGGTCTGTCGACGGGAGG-3'
SM020 binds to codons 100m to 98 (Sal I site underlined): 5'-CCTCCCGTCGACACACCGTGGCCACATTCATAATASNNAGCGTAG-3'
VHBACK(A4) binds in PelB leader signal of pHEN4 and beginning of cAb-Lys3 codons 1 to 4 (Sfi I site is underlined):
5'-CATGCCATGACTCGCGGCCCAGCCGGCCATGGCCGA(G/T)GT(G/C)CAGCT-3'

After digestion of both PCR fragments with Sal I, ligation and final PCR with VHBACK(A4) and AM006 primers a DNA fragment is generated which can be cloned in the pHEN4 cut with Sfi I and Bst EII (The Bst EII site occurs naturally in framework 4 of all VHH gene segments). The resulting phasmid DNA encodes a cAb-Lys 3 with Ser100a randomized and in which codons
Leu100i.Ser100j.Thr100k
CTT TCC ACT
CTG TCG ACT
of the cAb-Lys3 are mutated. These silent mutations harbours the restriction enzyme site for Sal I (underlined) and can be used for subsequent cloning or clone characterization. Also the nucleotides of the codons for
Cys100e.Gly100f.His100g
TGT GGT CAC
TGT GGC CAC,
are substituted. This silent mutation introduces a Bal I site (underlined) which, like the Sal I site, can be used for cloning or clone selection.

Using this strategy some 10,000 individual clones were generated of which some 24 individual clones were toothpicked and grown individually and tested separately for expression level and binding to hen egg-white lysozyme. All clones contained a mutation at position 100a and the sites for Sal I and Mlu I. Only two were binding to the lysozyme although with slight a reduced affinity. These mutations have the Ser100a substituted by respectively a Pro and His. All other clones contained a different amino acid such as Arg, Leu, or Lys, etc... and were shown to be expressed but unable to bind the lysozyme to a reasonable extend.

This proves that it is possible to mutate the loop with retention of the expression level but with an altered affinity/specificity towards lysozyme compared to the original cAb-Lys3.

### EXAMPLE 3

### Complete randomization of the 'TOT' loop.

With the oligo's VHBACK(A4) and SM021 a DNA fragment was generated by PCR on the cAb-Lys3 template which can be cloned after Sfi I/Sal I digestion into the pHEN4-aLys3 mutant digested with the same set of enzymes. The sequence of SM021 (Sal I site underlined) binds to codons 100m to 100e and from 96 to 92:
5'-CCTCCCGTCGACAGACCGTGGCCACA(SNN)₆CGAATCTGCCGCAC-3' These plasmids have their codons coding for Thr97 up to Glu100d removed and replaced by random codons (NNS)₆.

After construction of the library in a phage display vector such as the pHEN4 the proper binders are selected by panning with the elution/selection strategy explained in the description.

It is also possible to create a loop of different size. Then, the sequence of the primer SM021 was changed to one in which the random codons (NNS)ₓ is varied with X= 1 to 10 or more. The smaller X, the smaller the loop, and the larger X, the more extended the loop will be. These different libraries each with a different loop size are used to find the best fit for the clefts of the target proteins.

### EXAMPLE 4

### Veneering of the 'TUT' loop of cAb-Lys3

A slight different methodology of the loop randomization strategy generates a library in which the protruding loop from cAb-Lys3 is changed only at its outer surface, while preserving (most of) the internal and loop-structuring amino acids. This strategy is referred to as 'veneering'. The strategy consists of changing the SM021 primer with an SM022 primer:
5'-CCTCCCGTCGACAGACCGTGGCCACASNNATA(SNN)₃GTA(SNN)₂CGAATCTGCCGCA C-3'
and to perform a PCR in combination with primer VHBACK(A4) with pHEN4-αLys3 as template. This primer anneals to the codons 100m to 92 and randomizes codons 97, 98, 100, 100a, 100b and 100d, all others are retained. The randomized codons code for amino acids which were found to be facing outward the loop. After digestion of the PCR fragment with Sfi I and Sal I a library was constructed into the pHEN4 vector digested with the same enzymes. After expression of the mutated cAb on phage virions the proper binders were selected by panning with the elution/selection strategy explained in Example 3.

By changing the primer SM022 with a similar primer in which the (SNN)₃ is exchanged by (SNN)ₓ (X=1 to 6 or more, but not 3) and following the above protocol a library is generated in which the tip of the protruding loop is shortened or extended compared to the original loop. Also the other randomized positions can be enlarged or shortened to create a 'knob' on the side of the protruding loop. These different libraries are used for panning with the target molecules and selection of optimal binders.

### EXAMPLE 5

### Modification of the basic recognition unit surrounding the protruding loop

Once the recognition unit with the 'TUT' motif is constructed, the affinity or specificity can be increased by (subtle) modifications of the basic recognition unit (or 'platform') around the 'TUT' loop. The three best sites for these modifications are located at the N-terminal end of the recognition unit, the loop around amino acids 72/75 and the CDR1 or CDR2 regions.
1. The N-terminal end of the VHH is close to the antigen binding loops. This site can be used as the site mentioned before but the inserted amino acids will not be constrained. It is rather a fusion product which will be obtained. Therefore this site is a good site for inserting whole domains and for constructing molecules with bispecificity.
2. Reshaping the 72/75 loop for increasing the antigen binding surface and the modulation of the affinity/specificity by inserting/introducing new functional features. This loop is a good site to introduce mutations (randomisations) as was observed in camel and llama VHH clones insertions and deletions of one or two amino acids at this position. This site can be extended by three amino acids while the folding is still retained as well as its antigen binding activity. The structure of the cAb-Lys3 in this region is also not very well visible because of residual flexibility in this region in the crystal. Therefore it is anticipated that this region is a proper place to accommodate deletion and insertions. The presence of the restriction enzyme site Bsp HI around codons 81 to 82a
   Leu.Met.Asn
   CTC.ATG.AAC
   in combination with an oligonucleotide allows for mutagenising this region with PCR based technology and standard cloning techniques.
   E.g. primer binding at codons 67 to 82b (Bsp HI site
   underlined):
   3'-AAGTGGTAGAGGGTT(XXX)ₓTTGTGCCACATAGACGAGTACTTGTCG-5'
   together with the VHBACK(A4) primer in a PCR reaction on pHEN4-αLys3 will generate a fragment which can be cloned into pHEN4-αLys3 digested with Bsp HI and Sfi I. The generated library will encode the cAb-Lys3 protein in which the codons 72 to 75 are substituted by (XXX)ₓ. Depending on the size of nature of the (XXX)ₓ codons between codons 72 and 75 can be introduced, deleted or substituted.

For example, the introduction of Arg Gly Asp at this location could turn the protein into an integrin, or the randomization or inclusion of cysteine or histidines in this region might allow the chelation of metals in this location to generate a metallo-protein. In case one wants to incorporate a subdomain or a domain of an enzyme at this position then it is suggested to analyse the structure of the 'new' protein to find the amino acids enclosing the wanted domain with an orientation and distance equivalent to the orientation and distance of amino acids 72 and 75 in the VHH. If the structure is unknown or no amino acid fulfills this requirement, then it is advisable to introduce a short linker peptide which will allow to span the difference so that no unwanted constraints are imposed on the inserted domain which would inhibit the folding, stability and function of the chimeric protein. Similarly, changing the CDR1 or CDR2 amino acids can be performed to increase the affinity and specificity of the recognition unit with 'TUT' motif.

### EXAMPLE 6

### Increase of lifetime of the recognition unit with 'TUT' motif

The small single domain proteins of the invention have a good tissue penetration, a good bio-distribution and a rapid clearance from blood. For some applications (virus neutralization) it is however beneficial to have a longer lifetime in blood. To increase the lifetime within the blood, clone the protein with the 'TUT' motif can be cloned upstream of the hinge, CH2, and CH3 domains of IgG1 such as the IgG1 of human. This can be done by standard cloning techniques. The BstEII site occurring in human and cAb-Lys3 or other camel and llama VHH gene segments is a good site for ligating the two gene fragments to each other. The pHEN4 expression vector can be used for bacterial expression of these constructs, whereas the pCDNA-3 vector can be used for final expression in mammalian cell lines. This is no problem as it is reported that such constructs (without the CH1 and light chains) are well expressed in both systems, and functional proteins are obtained in these systems.

### EXAMPLE 7

### Intrabodies

To interact with target proteins which are normally located and/or functioning at internal cellular positions, it is necessary to bring the small recognition unit with the 'TUT' motif inside this cellular compartment (cytosol, nucleus, endoplasmic reticulum, mitochondria or chloroplast). The transformation of the gene-segment of the small recognition unit with the 'TUT' motif behind a suitable promotor and/or localisation signal, or extended with the SKDEL codons for targeting in the endoplasmic reticulum allows for expression and direction of the designed molecule to the cell compartment at will.

The cAb-TT2 was cloned behind the CMV promotor and a chloroplast leader signal. Transformation of this construct in Tobacco plants showed that the constructs were expressed and functioning as measured by ELISA. 2 gram leafs of the transgenic plant are grinded in a mortar in 2.5 ml PBS, 20% glycerol, 300 µl of 10 mM PMSF (phenylsulfonylfluoride). After a desalting over PD10 gelfiltration column (Pharmacia) we use 100 µl extract, 10 µl extract + 90 µl water and twofold dilutions in an ELISA. As control a non-transformed plant was used. Coating of the microtiter wells is with 100 µl 6.5 µg/ml tetanus toxoid. Blocking is with 1% casein in PBS, and detection of bound plant cAb-TT2 is with rabbit anti-camel IgG and goat anti-rabbit alkaline phosphatase conjugate (Sigma); paranitrophenylphosphate is the substrate and reading is done at 405 nm after 15 minutes. Thus, our small recognition units with 'TUT' motif are useful for the development as intrabodies.

### EXAMPLE 8

### Pentido-mimics

Once a 'TUT' loop is found and characterized, it is possible to synthesise it chemically as a constrained peptide. This peptide binds specifically and with good affinity inside the cavity of the target protein as numerous contacts are made. The 11 amino acids (from Asp95 to Cys100e) account for a surface of approximately 500 Å² of contact with the lysozyme active site cleft. This amount is certainly sufficient for generating specific binders from oligopeptides. Thus, the synthesis of the oligopeptide such as 'CGDSTIYASYYEGS' (the underlined sequence is the protruding loop part of cAb-Lys3, and the two Cys at the extremities serve to constrain the peptide conformation by disulphide bond formation) is used to test the specific binding to the lysozyme enzyme. In a subsequent step it is possible by organic chemistry to synthesise peptide analogues based on this peptide with similar binding characteristics.

Characterised 'TUT's' binding to other molecular clefts such as those from enzymes or receptor molecules can be used to design suitable peptido-mimics according to this strategy.

### EXAMPLE 8A

### Production of monoclonal antibodies against cAb-TT2

The production of monoclonal antibodies (MAbs) was obtained by an intrafootpad injection of the cAb-TT2 (5 µgr) without tag in complete Freunds adjuvant into a 4-6 weeks old female BALB/cxC57/B/L,F1 mouse. after eight days the mouse is sacrified and the popliteal lymph nodes are removed. The cells are mechanically released and washed once in DMEM medium.

The myeloma partner cells (NSO) are maintained in log-phase growth in DMEM. The cells are washed once and counted. The lymph node cells and the NSO cells are mixed in a 5 to 1 ratio and fused with 50% polyethylene glycol (PEG 4000) in DMEM. The cells are pelleted by centrifugation and resuspended gently in prewarmed medium (DMEM-20% fetal calf serum containing hypoxanthine, aminopterin, thymidine and streptomycin and penicillin as antibiotics). The cells are transferred into 250 ml DMEM and dispensed in microplates at approximately 5x10⁴ cells per well. The cultures are incubated for ten days at 37°C, 5% CO₂.

After ten days, the emergence of hybrid clones recorded. A total of 227 colonies were observed and their supernatants are tested in an ELISA. Purified cAb-TT2 at 1 µg/ml in PBS) is adsorbed overnight to wells of microtiter plates. The plates are washed, blocked with 1% casein-PBS, and incubated with the culture supernatants of the hybridoma cells for one hour at 37°C. The wells are washed again with PBS-tween20 (0,1%) and incubated with goat anti-mouse immunoglobulin conjugated with alkaline phosphatese (Sigma) for an additional hour. After the final wash, the enzyme was detected with p-nitrophenyl phosphate dissolved in 1 M diethanolamine supplemented with 1 mM MgSO₄ and adjusted with HCl to pH 9.8. The color development is monitored at 405 nm.

Out of the 227 hybrid colonies about 10% (24) showed a positive response in ELISA. From these 20 were selected for further analysis. The isotype class and subclass typing showed that 20 clones were of the IgG isotypes (IgG1-type, IgG2a and IgG2b), whereas the remaining four belong to the IgM class.

Testing the reactivity of 20 selected IgG monoclonals with cAb-TT2 specificity against purified camel IgG1 (i.e. conventional camel antibodies with light chains), IgG2 or IgG3 (i.e. camel heavy-chain isotypes), three camel VHH with unknown specificities (cAb-VHH9, cAb-VHH16 and cAb-VHH21), or cAb-lys1, cAb-TT1 and cAb-TT2 indicated that strong responses are obtained with the cAb-TT2, the original antigen for raising the monoclonals. Two monoclonals (23G8 and 9A9) recognize weakly other VHH domains, as well as camel IgG1. the supernatant of hybridoma 18C11, 21A3 and 3G2 mAbs give an intermediate response (50% of maximum response) to camel IgG1 and mAb 15A11 recognizes two other non-related VHHs (cAb-lysl and cAb-VHH21) to a lower extend (20% of maximum binding). Hence most of the anti-cAb-TT2 mAbs appear to be monospecific for cAb-TT2 indicating a private idiotypic specificty.

### EXAMPLE 9

### Immunisations with the recognition units with 'TUT' motif

The cAb-TT2 intra-footpad injection in BALB/c mouse led to the generation of 24 hybridoma's (out of 227 hybrids tested) with binding activity against cAb-TT2. From all these 24 monoclonals only two could bind weakly to other camel VHH's, whereas the binding to the cAb-TT2 was strong for all tested clones. Therefore, the generated monoclonals are most likely anti-idiotypic monoclonals.

It is known from the experiments of Zanetti (Nature 355, 476 (1992)) that the amino acids present at the CDR3 of the VH are immunogenic and that it is possible to immunize a mouse against malaria with a protein construct obtained by inserting a Plasmodium epitope in the CDR3 of VH. In analogy, in the constructs mentioned the protruding loop on the small recognition units is expected to be a good site for inserting other loops for in vivo immunizations.

It was realized that once a 'TUT' against a particular enzyme or receptor molecule is identified, it can be used to generate monoclonal antibodies. It is expected that the anti-idiotypic monoclonals will mimic the catalytic site of the original enzyme or receptor. This strategy can be used to generate Abzymes as the 'TUT' replaces the 'transition state of the substrate' used to develop antibodies with catalytic activity. The design and synthesis of stable 'transition state of the substrate' is often difficult or impossible. This strategy would bypass these synthesis difficulties.

### EXAMPLE 10

### Structure analysis of cAb-Lys3 co-crystallized with its antigen lysozyme

This example is shown in figure 1 in the Article "Nature Structural Biology" Volume 3, No. 9 from September 1996.

### EXAMPLE 11

### Immunisation protocols

Four different dromedaries (camelus dromedarius) are used for immunisations with different antigens, or different amount of antigen.

### Dromedary 1

- antigens:: Bovine RNase A at 0.1 mgr
Carbonic anhydrase at 0.1 mgr
b-lactamase at 1 mgr
Lysozyme at 1 mgr

Hepatitis B surface antigen serotype Ay at 0.25 mgr are mixed in approximately 0.5 ml saline, together with an additional two plant enzymes in a volume of 2 ml.
- Day 0:: Take 20 ml serum
Inject antigen mixture emulsified with CFA
(equal volume), subcutaneous
- Day 7: Take 20 ml serum
- Day 14: Boost with antigen mixed in IFA, subcutaneous = tube DAY 14
- Day 21: Take 20 ml serum
- Day 28: Boost with antigen mixed in IFA, subcutaneous = DAY 28
- Day 31: Take 20 ml blood (for lympocyte prep).
- Day 35: Take 20 ml serum
- Day 54: Boost with antigen mixed in IFA, subcutaneous = DAY 54
- Day 57: Take 50 ml blood (for lymphocyte prep)
- Day 61: Take 50 ml serum

### Dromedary 2

- antigens :: Bovine RNase A at 1 mgr
Carbonic anhydrase at 1 mgr
Lysozyme at 1.4 mgr
a-amylase at 1 mgr
are mixed in approximately 0.25 ml saline.
- Day 0: Take 20 ml serum
Inject antigen mixed emulsified with CFA (equal volume), subcutaneous
- Day 7: Take 20 ml serum
Boost with antigen mixed in IFA, subcutaneous
- Day 14: Take 20 ml serum
Boost with antigen mixed in IFA, subcutaneous
- Day 21: Take 20 ml serum
Boost with antigen mixed in IFA, subcutaneous
- Day 28: Take 20 ml serum
Boost with antigen mixed in IFA, subcutaneous
- Day 31: Take 20 ml blood
- Day 35: Take 20 ml serum
Boost with antigen mixed in IFA, subcutaneous
- Day 42: Take 20 ml serum
Boost with antigen mixed in IFA, subcutaneous
- Day 49: Take 20 ml serum
Boost with antigen mixed in IFA, subcutaneous
- Day 54: Take 20 ml serum
Boost with antigen mixed in IFA, subcutaneous
- Day 57: Take 50 ml blood
- Day 61: Take 50 ml serum

### Dromedary 3

- antigens:: Bovine RNase A at 1 mgr
Carbonic anhydrase at 1 mgr
b-lactamase at 0.1 mgr
Lysozyme at 0.1 mgr
TAT at 0.5 mgr

Hepatitis B surface antigen serotype Ad at 0.25 mgr are mixed in approximately 2.7 ml saline.
- Day 0:: Take 20 ml serum, take 20 ml blood (for preparation of lymphocytes) Inject antigen mixture together with CFA (equal volume), subcutaneous
- Day 7: Take 20 ml serum
- Day 14: Boost with antigen mixture in IFA, subcutaneous
- Day 21: Take 20 ml serum
- Day 28: Boost with antigen mixture in IFA, subcutaneous
- Day 31: Take 20 ml blood (for lympocyte preparation)
- Day 35: Take 20 ml serum
- Day 54: Boost with antigen mixture in IFA, subcutaneous
- Day 57: Take 50 ml blood (for lymphocyte preparation)

### Dromedary 4

- antigen:: TAT
are mixed and used to generate neutralising antibodies. All injections are done intramusculary.
- Day 0: Take 20 ml serum,
Inject 0.12 mgr cocktail + 2 ml PBS + 2 ml IFA
- Day 2: Priming with 0.24 mgr cocktail + 2 ml PBS + 2 ml IFA
- Day 4: Priming with 0.36 mgr cocktail + 2 ml PBS + 2 ml IFA
- Day 7: Priming with 0.48 mgr cocktail + 2 ml 0.07 M sodiumphosphate + 2 ml 0.07 M CaCl₂
- Day 9: Take 20 ml serum
Priming with 0.96 mgr cocktail + 2 ml 0,07 M sodiumphosphate + 2 ml 0.07 M CaCl₂
- Day 11: Priming with 1.50 mgr cocktail + 2.5 ml 0.07 M sodiumphosphate + 2.5 ml CaCl₂
- Day 14: Priming with 1.98 mgr cocktail + 3 ml sodiumphosphate + 3 ml CaCl₂
- Day 16: Priming with 1.32 mgr cocktail + 2 ml sodiumphosphate + 2 ml CaCl₂
- Day 18: Priming with 1.74 mgr cocktail + 2.5 ml sodiumphosphate + 2.5 ml CaCl₂
- Day 21: Priming with 2.16 mgr cocktail + 3 ml sodiumphosphate + 3 ml CaCl₂
- Day 24: Priming with 1.80 mgr cocktail + 3 ml sodiumphosphate + 3 ml CFA
- Day 31: Take 20 ml blood and 20 ml serum
- Day 65: Boost with 0.54 mgr cocktail + 2 ml sodiumphosphate + 2 ml CaCl₂
- Day 72: Boost with 1.08 mgr cocktail + 2 ml sodiumphosphate + 2 ml CaCl₂
- Day 75: Boost with 1.62 mgr cocktail + 2.5 ml sodiumphosphate + 2.5 ml CaCl₂
- Day 79: Take 50 ml blood
- Day 82: Take 50 ml serum

### EXAMPLE 12

### Immune response in function of time

Camel 2 (D2) has been injected with different antigens, as described in example 11. Blood was collected and serum was removed after coagulation.

Maxisorb plates were coated overnight at 4°C, respectively with, as follows:
- Lysozyme (3 µg/ml in PBS)
- Carbonic anhydrase bovine erythrocytes (4µg/ml in PBS)
- Pig pancreatic α-Amylase (3µg/ml in PBS)
- RNase A

The procedure for immobilization of the enzyme included 30 minutes pretreatment of the Maxisorb plate with 0.25% gluteraldehyde at room temperature. After washing with water, RNaseA was then added at 10µg/ml in PBS and further incubated overnight at 4°C.

Plates were blocked for at least 2hrs at room temperature with 1% casein in PBS.

The sera were diluted in 0.1% casein/PBS. 100µl of the diluted sera were added to the individual wells and incubated for 1hr at room temperature.

Bound IgG's were detected with a rabbit polyclonal anti camel IgG serum (I/1000 in 0.1% casein/PBS) followed by a goat-anti-rabbit IgG Alkaline Phosphatase conjugate (1/1 000 dilution in 0.1% casein/PBS). Between each step the wells were washed 5x with 200µl PBS/0.1%Tw20.

100µl p-nitro-phenyl-phosphate at 2mg/ml in ELISA buffer (10% diethanolamine buffer pH9.8 containg 0.5mM MgCl₂,) was added and OD at 405nm was measured after 20 minutes with Labsystems Multiscan RC ELISA plate reader. Optical densities were not corrected for background.

### EXAMPLE 13

### Solid-phase binding of fractionated leG of D2/54

### 1. Fractionation of IgG

1 ml of serum of camel day 54 was fractionated on ProteinG/A. Protein concentrations were determined spectrophotometrically at 278nm, assuming a ε_{1%}=13.5.

### 2. Coating of Maxisorb plates

Coating was performed overnight in the cold room with respectively:
- Lysozyme Sigma L6876 (3 µg/ml in PBS)
- Carbonic anhydrase bovine erythrocytes Sigma C3934 (4µg/ml in PBS)
- Pig pancreatic α-Amylase A6255 (3µg/ml in PBS)
- RNase A.

The procedure for immobilization of the enzyme included 30 minutes pretreatment of the Maxisorb plate with 0.25% gluteraldehyde. After washing with water, RNaseA was then added at 10µg/ml in PBS and further incubated overnight at 4°C.

Plates were blocked for at least 2hrs at room temperature with 1% casein in PBS.

### 3. Detection of bound IgG

Purified IgG's were individually tested on the individual immobilized antigens in the range 5000-39ng/ml. Dilution were made in 0.1% casein/PBS.

100µl of the diluted antibody solution was added to the individual wells and after 1hr incubation, bound IgG's were detected with total rabbit serlun anti-camel IgG-home made and subsequently with anti-rabbit-Alkaline Phosphatase conjugate (Sigma n° 8025). These reagents were diluted in 0.1% casein/PBS and were used at a 1:1000 dilution. Between each step the wells were washed 5x with 200µl PBS/0.1%Tw20.

Finally 100µl p-nitro-phenyl-phosphate at 2mg/ml in ELISA buffer (10% diethanolamine buffer pH9.8 containg 0.5mM MgCl₂) was added and OD at 405nm was measured after 10 minutes with Labsystems Multiscan RC ELISA plate reader.

Optical densities were not corrected for background.

### EXAMPLE 14

### Some epitopes of camel heavy chain IgGs are cavities

In order to demonstrate that heavy-chain IgG with a long CDR3-loop bind preferably to cavities, canyons or clefts present on the surface of native protein, some binding experiments were carried out.

As active sites of enzymes are preferably situated in the largest cleft, the heavy-chain antibodies are especially suited for development of inhibitors.

From binding experiments with as well α-amylase or carbonic anhydrase in the presence or absence of competitive inhibitors, it appeared that a substantial fraction of the heavy chain IgGs bind to the active site.

### 1. Bovine Pancratic α-amylase

Binding to solid phase enzyme of fractionated IgG1, IgG2a, IgG2b and IgG3 (range 2500-19.5 ng:ml) in the presence or absence of 1 mM Acarbose (pseudoheptasacharide with Ki 10⁻⁶M).

Bovine pancreatic α-amylase was coated overnight at 1.5µg/ml in PBS on Maxisorb plates at 4°C. Plates were blocked with 1% casein in PBS. Bound camel immunoglobulins were detected with rabbit anti-camel antiserum (R17 1/1000 dilution), followed by goat anti-rabit AP-conjugate (Sigma 1/1000 dilution). Between each step the wells were washed 5x with 200µl PBS/0.1%Tw20.

Finally 100µl p-nitro-phenyl-phosphate at 2mg/ml in ELISA buffer (10% diethanolamine buffer pH9.8 containg 0.5mM MgCl₂) was added and OD at 405nm was measured after 10 minutes with Labsystems Multiscan RC ELISA plate reader. Optical densities were not corrected for background.

From these experiments it can be concluded that a substantial portion of the amylase specific heavy chain antibodies bind to or close to the active site of the enzyme. Even more important, is the observation that the binding of IgG1 subclass to the antigen, is not affected by the inhibitor.

### 2. Bovine erythrocyte carbonic anhydrase

Binding to solid phase enzyme of fractionated IgG1, IgG2a, IgG2b and IgG3 in the presence or absence of 1mM dorzolamide (competitive inhibitor with Ki in the nanomolar range).

Carbonic anhydrase was coated overnight at 4µg/ml in PBS on Maxisorb plates at 4°C. Plates were blocked with 1% casein in PBS. Bound camel immunoglobulins were detected with rabbit anti-camel antiserum (R17 1/1000 dilution), followed by goat anti-rabit AP-conjugate (Sigma 1/1000 dilution). Between each step the wells werewashed 5x with 200µl PBS/0.1%Tw20.

Finally 100µl p-nitro-phenyl-phosphate at 2mg/ml in ELISA buffer (10% diethanolamine buffer pH9.8 containg 0.5mM MgCl₂) was added and OD at 405nm was measured after 10 minutes with Labsystems Multiscan RC ELISA plate reader. Optical densities were not corrected for background.

From these experiments it can be concluded that active site binders for this enzyme are only present in the IgG3 subclass.

### EXAMPLE 15

### Inhibition of pancreatic amylase by VHH of D2/61 IgG3

Based on the observation that the competitive inhibitor acarbose was able to compete with the binding of heavy-chain antibodies to solid-phase enzyme the following experiment was carried out which demonstrates that part of these antibodies inhibit the enzymatic activity of the enzyme. To rule out immunoprecipitation as cause of reduced enzymatic activity, as to expect the fractionated antibodies to be polyreactive and polyclonal, VHH fragments of the IgG3 fraction were prepared.

These VHH from the IgG3 fraction of D2/61 were generated by treatment with S.aureus V8 Endoglu-proteinase (Boehringer) in 0.1M ammoniumbicarbonate pH8 (1/50w/w enzyme/protein) for 2hrs. The efficiency of cleavage was followed by SDS-PAGE. After dialysis against PBS non-digested material and Fc-fragments were removed by protein G chromatography. The flow-through of the column contained the VHH fragments (see example 16).

Residual enzymatic activity was determined using the Ecoline® 25 Amylase assay (Merck-CNPG3 Method). The ready-to-use substrate solution was diluted 10-fold with PBS to lower the KSCN concentration to 90mM in order to avoid chaotrope induced dissociation.

Porcine pancreatic α-amylase (Sigma A-6255) was diluted in 0.1% casein PBS to a concentation of 1.5 µg/ml and 50 µl of this solution was incubated with 100µl of the purified VHH fragment (protein concentration 200µg/ml). After preincubation for 60 minutes the enzymatic activity was determined by adding part of the mixture to the 10-fold diluted substrate solution. The enzymatic activity was calculated from the increase in OD405 nm during 5 minutes. The enzymatic activity was reduced to 65%, relative to the enzymatic activity measured in the absence of VHH fragments, thus demonstrating that inhibitory antibodies are present.

### EXAMPLE 16

### Digestion and purification of VHH of IgG3 D2/61

VHH from the IgG3 fraction (1.72mg/ml) of camel 2 bleeding day 61 (D2/61) were generated by treatment with S.aureus V8 Endoglu-proteinase in 0.1M ammoniumbicarbonate pH8 (1/50w/w enzyme/protein ratio) for 2hrs. The efficiency of cleavage was followed by SDS-PAGE. After dialysis against PBS non-digested material and Fc-fragments were removed by protein G chromatography. The flow-through of the column contained the VHH fragments. The protein concentration of the VHH top fraction VHH (200µg/ml) was determined spectrofotometrically assuming a E1%=20. This fraction was used for inhibition assays.

Digestion lgG3 fraction of D2/61 with V8 S.aureus protease at pH8 in 0.1 M NH₄HCO₃ at 1/50 w/w enzyme/protein ratio for 2 hrs.
1. Molecular weight markers
2. Undigested IgG3
3. Digestion after 2hr with Endo Glu-protease V8 4-5-6. Frow-through of ProteinG-Sepharose column. 7-8-9. Elution of ProteinG-Sepharose with Glycine/HCl pH 2.7

### EXAMPLE 17

### Preparation of periferal blood lymphopytes

4 Dromedaires are used. Approximately 7 ml of blood (in EDTA) from each dromedary is collected and transported at 4°C. The blood is diluted with the same volume of sterile PBS and layered on top of 50 ml tubes (Wak chemie). The tubes are spun at 1000 g for 20 minutes (2200 rpm) at 20°C.

The liquid above the grid is transferred to a 50 ml Falcon tube (To eliminate the blood platelets, it is better to remove the supernatant and collecting only the lymphocytes which are banding just above the grid).

The cells are spun down at 2500 rpm for 15 minutes at 4°C. The pellet is resuspended in 0.5 ml PBS. After dilution of a 10 µl aliqout in 300 µl PBS the cells are counted. Each fraction contained approximately 3. 10⁷ cells/ml, of which only a minority were red blood cells. 5 tubes of 100 ml each were aliquotated in Eppendorf tubes and spun down at 2500 rpm, 5 min. The supernatant is removed and the pellet is frozen at -80°C. Each tube contains approximately 3. 10⁶ cells.

### EXAMPLE 18

### VHH library construction from peripheral blood lymphocytes and panning

### mRNA preparation

The frozen lymphocytes (2 tubes, each 5x 10⁶ lymphocytes/tube) collected from dromedary 2 (D2) at day 54, were used to isolate mRNA with the Micro-FastTrack Kit (Invitrogen). The mRNA was eleuted from the oligo-T solid support in 20 µl water. A total yield of 1.5 µg mRNA was obtained as measured spectrophotometrically (OD260 nm of 1 equals 35 µgr mRNA/ml).

### cDNA preparation

The cDNA was prepared from 1.5 µgr mRNA with the cDNA Cycle Kit (Invitrogen) according to the kit manufacturer recommendations. The cDNA was purified by phenol/chloroform extraction and by ethanol precipitation. The cDNA was resuspended in a total volume of 100 µl water.

### PCR amplification of VHH

The VHHs were amplified using 1 µl of the cDNA sample which is used as template in a PCR, using two gene specific primers CH2FORTA4 and an equimolar mixture of primers SM017 and SM018, in a total volume of 100 µl with 2.5 units Taq Polymerase (Boehringer) in the supplied buffer. Denaturation was at 94°C for 1 minute, annealing at 55°C for 1 minute and elongation at 72°C for 1 minute. This cycle was repeated 35 times.
CH2FORTA4:
5'-CGCCATCAAGGTACCAGTTGA-3'
SM017:
5'-CCAGCCGGCCATGGCTGATGTGCAGCTGGTGGAGTCTGG-3'
SM018:
5'-CCAGCCGGCCATGGCTCAGGTGCAGCTGGTGGAGTCTGG-3'

The most abondant amplification product had a size between 360 and 420 bp as visualised after gelelectrophoresis on 1.0% agarose gel in TBE and 0.5 µgr ethidium bromide/ml.

This PCR product was used as template for a reamplification with nested PCR primers A4SHORT (containing a SfiI site, underlined, the 15 nucleotides at its 3' end overlap with the 15 nucleotides at the 5' end of SM017 and SM018) and FRWRK4FOR (Not I site underlined).
A4SHORT:
5'-CATGCCATGACTCGCGGCCCAGCCGGCCATGGC-3'
FRWRK4FOR:
5'-GGACTAGTGCGGCCGCTGGAGACGGTGACCTGGGT-3'

The amplification product of 20 tubes was mixed and purified by Geneclean (Bio 101, Inc.), and digested overnight at 37°C with 50 units Not I and 50 units Sfi I (Gibco-BRL) in a total volume of 200 µl. The digested material was purified again by Geneclean.

### pHEN4 vector preparation.

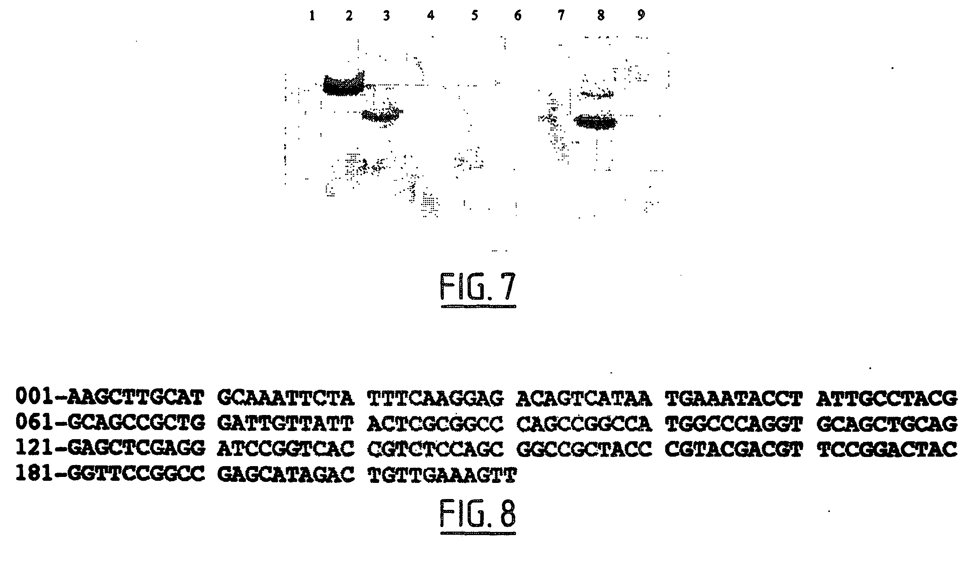

The region around the multiple cloning site of pHEN1 phagemid vector (Hoogenboom et al., Nucleic acid Reseach, 19, 4133-4137, 1992) was modified, so that it now contained a SfiI and NcoI site in the pe1B leader signal, and a Not I site preceding the hemagglutinin tag of (Mullinax et al., Proc. Natl. Acad. Sci. USA 87, 8095-8099, 1990) (figure 8).
HindIII:1 SfiI:87 NcoI:98 PstI:115 BamHI:129 BstEII:135 NotI:149
PelB leader signal:40-105
HA-tag: 157-186
gen pIII: starts at 199

The phagemid (40 µgr) was cleaved overnight with Sfi I and Not I. The cloning vector was purified by agarose gelelectrophoresis and Geneclean. The cut pHEN4 was eluted from the glassmilk (Geneclean) with 40 µl water.

### VHH-vector ligation.

The purified vector digested with Sfi and Not, and the purified VHH Sfi-Not fragment were put on agarose gel to estimate the concentration of the samples by ethidium bromide fluorescence. Based on these estimations, 40 µl (20 µg) of vector and 40 µl of VHH (5 µg) were mixed (expected molar ratio of 1/4) and ligated overnight at 16°C in a total volume of 100 µl, in 1x ligation buffer and 30 units T4 DNA ligase (Boehringer). The DNA was thereafter purified by phenolization and ethanol precipitation in the presence of 0.4 M LiCl. The DNA pellet was washed with 70% ethanol, dried and finally resuspended 100 µl water.

### Electrocompetent cells.

For the preparation of electrocompetent cells a preculture of an isolated TG1 E.coli colony on minimal medium plate was initiated. 1 ml of this preculture was transferred into 100 ml 2xTY medium supplemented with MgS0₄ and grown at 18°C until an OD of 0.5 (600 nm) was reached. The cells were harvested by centrifugation (3000 rpm, 10 min) and washed several fold (at least 5 times) with water. The final cell pellet was resuspended in 1 ml of 7% DMSO and aliquots of 50 µl were stored at -80°C until further use. A transformation efficiency of more than 5x 10⁸ /µgr pUC was obtained.

### Transformation, and library construction.

An aliquot of 1 µl of the ligated DNA sample was added to 50 µl electrocompetent TG1 cells in 2 mm electroporation cuvettes (EUROGENTEC, Belgium) kept on ice. After electrotransformation (2.5 kV, 25 µF, 200 Ohm), the cells are immediately brought into 1 ml SOC medium and incubated at 37°C for 1 hour. Seventy of these tubes were mixed and plated on a total of 50 large (24.3 cm x 24.3 cm) LB agar plates containing 100 µgr ampicillin/ml to select for the transformed cells and incubated overnight at 37°C. At least 5x 10⁶ individual transformants were obtained and these were scraped from the plates with 2xTY medium, washed with 2xTY by centrifugation and finally resuspended in 100 ml 2xTY, 100 µg/ml ampicillin, 1% glucose and 50% glycerol. The bacterial suspension was frozen at -80°C until further use.

### M13K07 helper phase preparation

A preculture of E.coli cells containing M13K07 is used to inoculate 1 litre 2xTY medium, supplemented with 70 µgr/ml kanamycin, and is incubated overnight at 37°C with vigourous shaking. The bacteria are removed by two centrifugations (15 minutes, at 8000 rmp). The bacterial cells remaining in the supernatant are heat inactivated by a 30 minutes incubation at 55°C. The supernatant is filtrated through a 0.2 µ filter. The phages can be concentrated by PEG precipitation. To this end 1/5 volume of 2.5 M Nacl, 20% PEG 8000 (200 ml) is added to the 1 litre supernatant, and the mixture kept on ice for at least 1 hour.

The sample is centrifugated for 40 minutes at 5000 rpm or 15-20 minutes at 13000 rpm. The M13K07 pellet is resuspended in sterile PBS (10 ml). The concentration of the phages can be determined spectrophotometrically (OD 1 at 260 nm corresponds to 4 x 10¹⁰ phages/ml), or the titer can be determined by adding serial dilutions in 10 mM MgC12 to exponentially growing TG1 cells and plating the cells on LB plates containing 70 µg/ml kanamycin. (M13K07 carries the Kanamycin resistance gene). The phages are brought to a titer of at least 10¹² phages/ml.

### Phage rescue and panning

### 1. Phage rescue

The cells transformed or carrying the pHEN4 recombinants are grown in 2xTY, ampicillin (100 µg/ml), 1% glucose. The cells are pelleted once the culture reaches an OD of 0.6 (600 nM). The cell pellet is washed in 2xTY medium and resuspended in the same medium supplemented with ampicillin (100 µgr/ml). The cells are infected with M13K07 at a multiplicity of infection of 10 to 20. After an incubation period of 20 minutes at room temperature, the cell suspension is bought to 70 µgr kanamycin/ml, and incubated overnight at 37°C with vigourously shaking.

The virus particles and virions are purified by first removing the bacterial cells through a centrifugation step (5000 rpm, 15 minutes) and filtration through a 0.4 or 0.2 µm filter. The phages are precipitates by addition of 1/4 volume of PEG solution (20% PEG, 2.5M NaCl), and incubation on ice for at least one hour. The phages are pelleted by 30 minutes centrifugation at 15,000 rpm. Occasionally an additional precipitation step was included by resuspension of the phages in approximately 1 ml PBS and adding 0.25 ml PEG solution. After incubation on ice for 30 minutes the phages can be pelleted by centrifugation 10 minutes, 13,000 rpm in an Eppendorf centrifuge. The phages are resuspended in PBS (100 µl). The concentration of the phages is measured by UV absorption (260 nm), OD of 1 corresponds to a phage concentration of 22 x 10¹⁰ phages/ml or a concentration of 44 x 10¹⁰ phagemid virions/ml. The phages/phagemids are brought to a concentration of 10¹²/ml with PBS, 0.1% casein and used for panning.

### 2. panning

Two methods were used for panning. In one method Nunc immunotubes (Nunc maxisop, startubes) were used to coat the antigens overnight at 4°C (1 ml amylase (100 µg/ml PBS), or 1 ml carbonic anhydrase (100 µg/ml PBS), 1 ml lysozyme (200 µg/ml PBS), or 1 ml RNase A (100 µg/ml TBS/CaCl₂ in 0.25% glutaraldehyde)). The tubes were washed 10 times with sterile PBS, before incubation with the rescued virions. After one hour incubation the non-bound virions and phages are removed by at least 10 washes with sterile PBS, Tween. The bound virions and phages are eluted by adding 1 ml triethylamine (0.1 M), and incubation for 5 minutes at room temperature, neutralized with 0.5 ml 1 M Tris pH 7.4 2 ml of exponentially growing TG1 cells are added and after an incubation period of 20 minutes the cells are plated on LB/ampicillin plates. The next day the colonies are scraped from the plates and can be used for the next round of panning after rescue with M13K07.

For the second method 4 wells were used of a microtiter plate for immobilizing the antigens (as above but with 100 µl volume/well). Washing of the wells, incubation with phages/phagemids and elution, neutralization and TG1 infection is as described above. Background is measured by adding virus particles in wells which are only coated with the blocking agent (1% Casein in PBS). The results for the four different antigens were:

| 1 st round | INPUT | ELUTED | BACKGROUND |
|---|---|---|---|
| amylase | 4x10¹⁰ | 0.06x10⁶ | 0.025x10⁶ |
| carbonic anhydrase | 4x10¹⁰ | 0.06x10⁶ | 0.025x10⁶ |
| lysozyme | 4x10¹⁰ | 0.1x10⁶ | 0.025x10⁶ |
| RNase Ae | 4x10¹⁰ | 0.06x10⁶ | 0.025x10⁶ |

| 2nd Round | INPUT | ELUTED | BACKGROUND |
|---|---|---|---|
| amylase | 4x10¹¹ | 1.3x10⁶ | 0.27x10⁶ |
| carbonic anhydrase | 4x10¹¹ | 1.3x10⁶ | 0.056x10⁶ |
| lysozyme | 4x10¹¹ | 0.26x10⁶ | 0.2x10⁶ |
| RNase A | 4x10¹¹ | 1.3x10⁶ | 0.048x10⁶ |

| 3rd Round | | | |
|---|---|---|---|
| amylase | 4x10¹¹ | 2.8x10⁶ | 0.08x10⁶ |
| carbonic anhydrase | 1x10⁸ | 0.05x10⁶ | 0.008x10⁶ |
| lysozyme | 1x10¹¹ | 0.5x10⁶ | 0.016x10⁶ |
| RNase A | 1x10¹¹ | 2.8x10⁶ | 0.004x10⁶ |

### Selection of individual binders

After the last round of panning, the antigen binders are selected by chosing randomly 24 individual clones from the plate and growing the cells in 2xTY with 100 µgr ampicillin/ml. Two protocols were used to detect the presence of antigen binding VHH. Either the VHH expression was induced with 1 mM IPTG when the cells reached the exponential growing phase, or the cells were infected with M13K07 helper phage. In the former strategy, the antigen binding capacity of the cAb could be checked in an ELISA of the culture supernatants with anti-HA-tag monoclonal (clone BBBB BAbCo). In the second strategy the virions having antigen binders on their tip are screened by ELISA with the anti-M13 detection kit (Pharmacia).

In the ELISA experiment or the phage ELISA, we showed that 23 out of the 24 clones from the carbonic anhydrase pannings were binding to the carbonic anhydrase. These clones were numbered CA01 till CA24. for the RNAseA pannings, all 24 clones scored positive, these are referred to as RN01 till RN24. Plasmid DNA from clones RN01 till RN12 was prepared and the insert was sequenced. RN02 and RN06 are identical and RN06 was taken as reference. All other clones are identical to the RN05 clone which was taken as reference for the second set.

For the carbonic anhydrase 12 clones (CA01 - CA12) were sequenced. The sequence of CA01=CA06=CA07=CA09=CA12, the clones CA04 and CA10 were unique and clones CA02, CA03, CA05, CA08 and CA08 were identical with the exception of the presence of a silent mutation in the CDR3 for CA05 and a different first amino acid (which was forced by the PCR primer). So there occurred at least 4 different set of clones of which CA04, CA05, CA06, CA10 are taken as the reference clones.

The nucleotide acid sequence of CA04 and CA05 is given in example 19. It can be seen that both the CA04 and the CA05 clone are indeed a VHH originating from a heavy chain antibody and not from a conventional antibody (with light chain). The presence of key markers Ser11 (codon 31-33nc), Phe37 (codon 109-111nc), Glu44-Arg45 (codons 130-135nc) and Gly47 (codon 139-141nc) proves this statement. The presence of a possible disulfide bridge between CDR1 and CDR3 in both cases as indicated by the presence of additional Cysteines (codons 97-99nc, and codon 313-315 for CA04 or 319-321nc for CA05) is also frequently observed in camel VHHs. The long CDR3 of 18 amino acids for CA04 (codons 295-348nc) and of 19 amino acids (codons 289-345nc) for CA05 shows that both cAbs have a long third hypervariable loop similar to that of cAb-lys3.

It will be shown in example 18a that CA04 binds into the active site of the carbonic anhydrase, while CA05 does not. This does not mean that the long loop of CA05 fails to bind into the grooves of the antigen, as it is known from the crystal structure of carbonic anhydrase that the active site for this enzyme is only the second largest groove of the enzyme. The largest is located at the other end from the active site, and it might be that the CA05 long CDR3 loop binds into this groove.

Three methods were used for panning. In the first method Nunc immunotubes (ref) are used to coat the antigens (amylase, carbonic anhydrase, lysozyme and RNase A). The tubes were washed 10 times with sterile PBS, before incubation with the rescued virions. After a one hour incubation the non-bound virions and phages are removed by at least 10 washes with sterile PBS, Tween. The bound virions and phages are eluted by adding 1 ml triethylamine (0.1 M), and incubating for 5 minutes at room temperature, neutralised with 0.5 ml 1 M Tris pH 7.4. 2 ml of exponentially growing TG1 cells are added and after an incubation period of 20 minutes the cells are plated on LB/ampicillin plates. The next day the colonies are scraped from the plates and can be used for the next round of panning afer rescue with M13K07.

For the second method 4 wells were used of a microtiter plate for immobilising the antigens. Elution is with 100 µl triethylamine.

### EXAMPLE 18a

### Binding of the camel single domain antibody CA04 into the active site of carbonic anhydrase

All 24 clones isolated after panning with carbonic anhydrase were induced with 1 mM IPTG. The expressed camel single domain VHHs (cAbs) were extracted from the periplams and used in an ELISA experiment in which the carbonic anhydrase was immobilised in the wells of the microtiter plate. The periplasmic extracted proteins (100 µl) were incubated in the presence of 50µl PBS, or 50 µl of a 2% solution dorzolamide (TRUSOPT^{R}), or a 50 µl zcetazolamide soultion (DIAMOX^{R}-Cyanamid). The two latter drugs are binding into the active site of the carbonic anhydrase. After 1 hour incubation, the wells are washed with PBS, Tween, incubated with 1/5000 BABCO anti-HA antibody in 0.1% casein, PBS for 1 hour at room temperature, washed and incubated with Rabbit anti-mouse alkaline phosphatase conjugate (Sigma) at a 1/1000 dilution. Substrate is pare nitro phenyl phosphate (2mg/ml) and readings were done after 10 minutes at 405 nm. (table)

| CLONES\INHIBITOR | NONE | Dorzolamide | |
|---|---|---|---|
| Acetazolamide | | | |
| CA01 | 0.75 | 0.143 | 0.17 |
| CA02 | 1.04 | 0.85 | 0.90 |
| CA03 | 1.04 | 0.86 | 0.94 |
| CA04 | 0.74 | 0.22 | 0.26 |
| CA05 | 0.85 | 0.75 | 0.77 |
| CA06 | 0.62 | 0.25 | 0.27 |
| CA07 | 0.87 | 0.23 | 0.28 |
| CA08 | 1.22 | 1.06 | 1.120 |
| CA09 | 0.83 | 0.17 | 0.23 |
| CA10 | 0.68 | 0.64 | 0.64 |
| CA11 | 1.00 | 0.93 | 0.92 |
| CA12 | 0.79 | 0.14 | 0.18 |
| CA13 | 0.89 | 0.15 | 0.19 |
| CA14 | 0.68 | 0.13 | 0.30 |
| CA15 | 0.22 | 0.12 | 0.14 |
| CA16 | 0.88 | 0.46 | 0.47 |
| CA17 | 0.48 | 0.12 | 0.13 |
| CA18 | 0.73 | 0.13 | 0.17 |
| CA19 | 0.74 | 0.13 | 0.17 |
| CA20 | 0.74 | 0.13 | 0.17 |
| CA21 | 0.84 | 0.15 | 0.20 |
| CA22 | 0.84 | 0.15 | 0.19 |
| CA23 | 1.04 | 0.99 | 1.01 |
| CA24 | 1.13 | 1.09 | 1.15 |

Clone CA15 is not binding to carbonic anhydrase, or is a weak binder.

Clone CA16 is only partially displaced by both dorzolamide and acetazolamide.

Binding of cAb CA02, CA03, CA05, CA08, CA10, CA11, CA23 and CA24 is not displaced by the active site binding drugs.

The cAbs of clones CA01, CA04, CA06, CA07, CA09, CA12, CA13, CA14, CA17, CA18, CA19, CA20, CA21, CA22 are displaced by both the dorzolamide and the acetazolamide. These cAbs are therefore considered as active site binders. The ratio of active site binders is 14 out of 24 clones. From the sequencing data of the CA01 to CA12 we know that there are at least two different groups (CA04, CA06) among the active site binders.

### EXAMPLE 19

### Recloning and expression of binders with His6 tag and cAb-characterization

### Recloning in pHEN6.

The HA tag and M13 pIII gene between the NotI and Eco RI gene of pHEN4 was replaced by six His codons. Within the Sfi I and Not I sites a cAb-Lys 3 gene was inserted (with the last Ser codon 'AGC' of the VHH replaced by 'TCACGC", this will introduce an additional Ser-Arg dipeptide). The following sequence is obtained (figure) for pHEN6-Lys3.

The plasmid pHEN6-Lys3 is digested with HindIII and Bst EII under optimal buffer and temperature conditions for the enzymes (Gibco-BRL). The cAb-Lys3 containing fragment is further cleaved with an additional digestion with NcoI. The linearised plasmid DNA is purified by phenolisation and ethanol precipitation in the presence of 0.4 M LiCl. The DNA is resuspended in 20 µl water, 3 µl is used to estimate the concentration by fluorescence in agarose gel and the remaining material is brought to a concentration of 100 ngr/µl.

The pHEN4-CA04 or the pHEN4-CA05 are similarly digested by HindIII and Not I. The cAb-CA04 and the cAb-CA05 containing fragment are purified from agarose gel with Geneclean. Approximately 100 ngr of these fragments (estimated from fluorescence in agarose gel) are mixed with 100 ngr of HindIII-Not I cut pHEN6 vector and ligated in a total volume of 10 µl with 2.5 units T4 DNA ligase (Boehringer) overnight at room temperature. The ligated DNA (2 µl) is mixed with electrocompetent WK6 cells, and plated on LB/ampicillin plates. The pHEN6-CA04 or pHEN6-CA05 containing colonies are screened by colony PCR with the universal forward and reverse sequencing primer (standard PCR conditions). Cutting the PCR fragment with Eco81I and separation of the resulting fragments on 5% acrylamide gel allows the identification and discrimination between residual pHEN6-Lys3 and pHEN6-CA04 orpHEN6-CA05 clones due to the larger CDR3 of the cAb-Lys3 insert.

The plasmids of the positively scored colonies were prepared with alkaline lysis method and used as a template for dideoxy-sequencing. The sequence of the pHEN6-CA04 and pHEN6-CA05 between the HindIII and Eco RI sites is given in the figures (the cAb-CA04 and cAB-CA05 are in bold, and the his6-tag is underlined).

### Protein expression and purification

An overnight culture of WK6 cells freshly transformed with plasmid pHEN6-CA04 and pHEN6-CA05 were used to inoculate 8 litre of TB medium containing 100 µgr/ml ampicillin and 0.1% glucose. After growth at 37°C and when the culture reached an absorbance of 0.75-1.0 at 600 nm, expression was induced by addition of IPTG to a final concentration of 1 mM and cell growth was continued for an additional 16 hours at 28°C. The periplasmic fractions were prepared essentially according to Skerra and Plückthun (Science 240, 1038-1041, 1988). Cells were harvested by centrifugation at 4000 g for 10 minutes at 4°C and resuspended in 1% of the original volume in icecold TES buffer (0.2 M Tris-HCl pH 8.0, 0.5 MM EDTA, 0.5 M sucrose). After one hour incubation on ice, the cells were subjected to a mild osmotic shock by the addition of 1.5% volume of ice-cold 1/4 diluted TES buffer. After one hour incubation on ice, the cells were centrifugated twice at 13000 g for 30 minutes at 4°C and PMSF (phenyl methyl sulfonyl fluoride) to a final concentration of 1 mM was added to the 200 ml of supematant which constituted the periplasmic fraction.

This periplasmic fraction was concentrated 10 fold by ultrafiltration in an Amicon cell (Millipore filter with MW cut off of 5kDa) before being bound on a 2 ml Ni-NTA affinity column (Qiagen). After washing with 40 ml of 50 mM sodiumphosphate buffer pH 8.0, 300 mM NACl, 10% glycerol buffer, the 6xHis tagged single domain antibody was eluted with a 40 ml linear gradient from 0 to 0.5 M imidazole in the same buffer. The fractions containing cAb-CA04 or cAb-CA05 respectively were pooled, concentrated 10 times by ultrafiltration and the imidazole was removed by passing over a Superdex-75 (Pharmacia) column using PBS buffer. 1.5 mgr pure protein was obtained (as measured spectrophotometrically at 280 nm) and concentrated by ultrafiltration to a concentration of 3 mg/ml.

### EXAMPLE 20

### Affinity measurement of CA04-HIS constuct by competitive ELISA

Transformed TG1 cell were induced with IPTG for the production of soluble protein. After harvesting the cells from 40 ml culture, the periplasmic fraction was prepared. In brief, the pellet was resuspended in icecold TES (1.2 ml 50mM TRIS pH 5mM EDTA, 20% sucrose) and incubated for 15 minutes on ice. After centrifugation the supernatant was removed and the pellet was resuspended in 1.2 ml of chilled water. The suspension was left on ice for another 30 minutes. After centrifugation at 14.000 rpm the supernatant was recovered and used in subsequent binding and competition assays.

As well for binding as competition assays Carbonic Anhydrase was coated on Maxisorb plates (Nunc) at a concentration of 1 µg/ml in PBS (100 µl overnight at 4°C). Plates were blocked with 200 µl 1% casein in PBS) for 2hr at room temperature. For the competition assay, mixtures of the supernatant at 1/100 dilution in 0.1% casein/PBS with free antigen varying in concentration between 1-10⁴nM were prepared. 100 µl of these mixtures were added to different wells of the plate. After 2hr bound CA04-HIS was detected with Histidine tag specific monoclonal antibody (Dianova, dia900, mouse monoclonal antibody IgG1, anti (His)₆ tag) and subsequently with rabbit anti-mouse alkaline phosphatase conjugate. Both secondary reagents were used at a dilution 1/1000 in 0.1% casein/PBS. The substrate (100 µl of 2mg/ml para-nitrophenolphosphate in ELISA buffer) was added and OD 405nm was measured after 15 minutes. From the plot of OD 405nm vs concentration of free antigen a Kd of 50nM was estimated.

### EXAMPLE 21

### Affinity measurement and kinetic analysis of the CA04:carbonic anhydrase interaction

The kon, koff and Kd of the CA04 carbonic anhydrase interaction were determined with an IAsys biosensor instrument.

An IAsys carboxymethyl dextraan cuvette (CMD) was used to follow the interaction. The antigen was immobilized on the cuvette by electrostatic absorption in the CMD matrix and by the subsequent covalent reaction of lysyl groups with activated carboxyl groups on the CMD polymer. Activation of the carboxyl groups was achieved by the EDC/NHS coupeling chemistry (Johnson et al), using a EDC/NHS coupeling kit (Affinity Sensors).

After a 7 min activation of the CMD cuvette, the cuvette was washed with 10mM NaAc buffer. 100µg/ml of carbonic anhydrase was added to the cuvette and allowed to react for 10 minutes. After washing the cuvette with PBS, the remaining activated carboxyl groups were subsequently deactivated by adding 1M ethanolamine pH 8.0. After deactivation, several washes with 10mM NaOH were performed to remove all carbonic anhydrase which was not covalently attatched. Calculation of the amount of immobilized antigen yielded a value of 6ng/mm². The stoichiometry of binding was measured by adding a saturating amount of CA04 to the cuvette and was equal to 0.4.

All experiments were performed in PBS at 27°C and at a stirr setting of 100. The regeneration conditions were optimized. A one minute wash with 10mM NaOH was used.

Binding traces for different concentrations of CA04 (2 10⁻⁸M to 1.5 10⁻⁷M) were performed in triplicate and allowed to go to equilibrium. The curves were fitted with a single exponential using FASTfit (Affinity Sensors). Baseline corrections were taken into account. The resulting pseudo-first order rate constants obtained from these fits were plotted against the concentration of CA04. The kon was determined by linear regression and yielded a value of 6.2 10⁵ M⁻¹s⁻¹. The value is set as a lower limit because of the occurance of mass transport limitations. This was seen by plotting derivative of the signal versus the signal for a high concentration of CA04 wich showed significant curvature.

Dissociation phases, where after addition of saturating amounts of CA04 the cuvette is washed with PBS, were followed in the presence of 0.6µM of carbonic anhydrase (in triplicate). The curves were fitted using the FASTfit software (Affinity Sensors). The curves were fitted to a double exponential in wich the slower phase was interpreted as being the result of rebinding while the faster one reflects the actual off-rate. This value is equal to 0.02s⁻¹.

Calculation of the Kd based on the kinetic analysis yields a value equal to 32nM.

The Kd value was also determined by plottin the equilibrium values versus the concentration of CA04 (3 10⁻⁸ to 1 10¹⁻⁷M) and fitted to a hyperbolic relationship again using FASTfit (Affinity Sensors). The Kd value obtained from this analysis was equal to 60nM.

### EXAMPLE 22

### Inhibition of bovine erythrocyte carbonic anhydrase by Ca04-His

Carbonic anhydrase (Sigma.C-3934) was dissolved in PBS and the protein concentration was determined spectrophotometrically at 280nm using a E1%=19.

The concentration of the purified CA04-His was determined spectrophotometrically using a calculated extinction coefficient of E1%=17 (PcGene). The enzyme was mixed at a fixed final concentration of 2.3µM with variable amounts of CA04-His (range 1-8µM) in a constant volume of 60 µl. After preincubation for 15 minutes at room temperature, 945 µl PBS and 5µl of para-nitro-phenylacetate (2% solution in absolute ethanol) were added (Pocker Y. and Stone J.T., Biochemistry, 6, 1967, 668-678). The reaction mixture was transferred immediately to a cuvette and the increase in OD405nm was monitored for at least 5 minutes at room temperature. The enzymatic velocities were corrected for spontaneous hydrolysis of the substrate. Residual activity was calculated relative to the enzymatic activity measured in the absence of CA04-HIS.

### EXAMPLE 23

### In vivo neutralization of tetanus toxin

The in vivo neutralization test of tetanus toxin is performed as described by Simpson et al., (J. Pharm. Exp. Therapeutics 254, 98-103, 1990). Sixty-four NMRI mice (male and female) of 8-12 weeks of age are randomly grouped in 8 groups (4 males and 4 females). The mice are injected i.p. with tetanus toxin (RIT, Smith Kline Beecham, Rixensart, Belgium), antibody fragments or both as follows:

| | |
|---|---|
| group 1 | PBS + cAb-TT1 |
| group 2 | PBS + cAb-TT2 |
| group 3 | PBS + Tetanus toxin (10xLD50) |
| group 4 | PBS + Tetanus toxin (10xLD50)+cAb-TT1 (4µg) |
| group 5 | PBS + Tetanus toxin (10xLD50)+cAb-TT1 (40µg) |
| group 6 | PBS + Tetanus toxin (10xLD50)+cAb-TT2 (4 µg) |
| group 7 | PBS + Tetanus toxin (10xLD50)+cAb-TT2 (40 µg) |
| group 8 | PBS + Tetanus toxin (10xLD50)+ non-specific |
| | cAb-VHH21 (40 µg) |

The total volume of injection is 0.1 ml in all cases. The mixture of VHHs and tetanus toxin is incubated for 30 minutes at room temperature before injection. The mice are followed for two weeks.

## Claims

1. A molecule which is a V_{HH} domain of a camelid heavy chain antibody comprising a CDR3 loop of a camelid species heavy-chain antibody, wherein said CDR3 loop protrudes from the antigen-binding site and recognizes and interacts with the active site residues of a biologically active target molecule,
provided said molecule is not a V_{HH} consisting in one of the following protein sequences:
i) αTT1: Glu-Val-Gln-Leu-Gln-Ala-Ser-Gly-Gly-Gly-Ser-Val-Gln-Ala-Gly-Gly-Ser-Leu-Arg-Leu-Ser-Cys-Ala-Ala-Ser-Gly-Gly-Gln-Thr-Phe-Asp-Ser-Tyr-Ala-Met-Ala-Trp-Phe-Arg-Gln-Ala-Pro-Gly-Lys-Glu-Cys-Glu-Leu-Val-Ser-Ser-Ile-Ile-Gly-Asp-Asp-Asn-Arg-Asn-Tyr-Ala-Asp-Ser-Val-Lys-Gly-Arg-Phe-Thr-Ile-Ser-Arg-Asp-Asn-Ala-Lys-Asn-Thr-Val-Tyr-Leu-Gln-Met-Asp-Arg-Leu-Asn-Pro-Glu-Asp-Thr-Ala-Val-Tyr-Tyr-Cys-Ala-Gln-Leu-Gly-Ser-Ala-Arg-Ser-Ala-Met-Tyr-Cys-Ala-Gly-Gln-Gly-Tlu-Gln-Val-Thr-Val-Ser-Ser
ii) aTT2: Glu-Val-Gln-Leu-Gln-Ala-Ser-Gly-Gly-Gly-Ser-Val-Gln-Ala-Gly-Gly-Ser-Leu-Arg-Leu-Ser-Cys-Thr-Ala-Ala-Asn-Tyr-Ala-Phe-Asp-Ser-Lys-Thr-Val-Gly-Trp-Phe-Arg-Gln-Val-Pro-Gly-Lys-Glu-Arg-Glu-Gly-Val-Ala-Gly-Ile-Ser-Ser-Gly-Gly-Ser-Thr-Thr-Ala-Tyr-Ser-Asp-Ser-Val-Lys-Gly-Arg-Tyr-Thr-Val-Ser-Leu-Glu-Asn-Ala-Lys-Asn-Thr-Val-Tyr-Leu-Leu-Ile-Asp-Asn-Leu-Gln-Pro-Glu-Asp-Tlu-Ala-Ile-Tyr-Tyr-Cys-Ala-Gly-Val-Ser-Gly-Trp-Arg-Gly-Arg-Gln-Trp-Leu-Leu-Leu-Ala-Glu-Thr-Tyr-Arg-Phe-Trp-Gly-Gln-Gly-Thr-Gln-Val-Thr-Val-Ser-Ser
iii) αLYS2: Glu-Val-Gln-Leu-Gln-Ala-Ser-Gly-Gly-Gly-Ser-Val-Gln-Ala-Gly-Gln-Ser-Leu-Arg-Leu-Ser-Cys-Ala-Thr-Ser-Gly-Ala-Thr-Ser-Ser-Asn-Ser-Cys-Met-Gly-Trp-Phe-Alg-Gln-Ala-Pro-Gly-Lys-Glu-Alg-Glu-Gly-Val-Ala-Val-Ile-Asp-Thr-Gly-Arg-Gly-Asn-Thr-Ala-Tyr-Ala-Asp-Ser-Val-Gln-Gly-Arg-Leu-Thr-Ile-Ser-Leu-Asp-Asn-Ala-Lys-Asn-Thr-Leu-Tyr-Leu-Gln-Met-Asn-Ser-Leu-Lys-Pro-Glu-Asp-Thr-Ala-Met-Tyr-Tyr-Cys-Ala-Ala-Asp-Thr-Ser-Thr-Trp-Tyr-Arg-Gly-Tyr-Cys-Gly-Tlu-Asn-Pro-Asn-Tyr-Phe-Ser-Tyr-Trp-Gly-Gln-Gly-Thr-Gln-Val-Thr-Val-Ser-Ser, and
iv) αLYS3: Asp-Val-Gln-Leu-Gln-Ala-Ser-Gly-Gly-Gly-Ser-Val-Gln-Ala-Gly-Gly-Ser-Leu-Arg-Leu-Ser-Cys-Ala-Ala-Ser-Gly-Tyr-Tlu-Ile-Gly-Pro- Tyr-Cys-Met-Gly-Trp-Phe-Arg-Gln-Ala-Pro-Gly-Lys-Glu-Arg-Glu-Gly-Val-Ala-Ala-Ile-Asn-Met-Gly-Gly-Gly-Ile-Thr-Tyr-Tyr-Ala-Asp-Ser-Val-Lys-Gly-Arg-Phe-Thr-Ile-Ser-Gln-Asp-Asn-Ala-Lys-Asn-Thr-Val-Tyr-Leu-Leu-Met-Asn-Ser-Leu-Glu-Pro-Glu-Asp-Thr-Ala-Ile-Tyr-Tyr-Cys- Ala-Ala-Asp-Ser-Thr-Ile-Tyr-Ala-Ser-Tyr-Tyr-Glu-Cys-Gly-His-Gly-Leu-Ser-Thr-Gly-Gly-Tyr-Gly-Tyr-Asp-Ser-Trp-Gly-Gln-Gly-Thr-Gln-Val-Thr-Val-Ser-Ser,
for use in therapy.

2. A molecule as defined in claim 1, for use in the neutralization of the biological function of said biologically active target molecule.

3. Use of a molecule which is a V_{HH} domain of a camelid heavy chain comprising a CDR3 loop of a camelid species heavy-chain antibody, wherein said CDR3 loop protudes from the antigen-binding site and recognizes and interacts with the active site residues of a biologically active target molecule for the manufacture of a drug for the treatment of pancreatic disorders.

4. A molecule which is a V_{HH} domain of a camelid heavy chain antibody comprising a CDR3 loop of a camelid species heavy-chain antibody, wherein said CDR3 loop protrudes from the antigen-binding site and recognizes and interacts with the active site residues of a biologically active target molecule, wherein said biologically active target molecule is a viral specific protein, for use in therapy.

5. A molecule which is a V_{HH} domain of a camelid heavy chain antibody comprising a CDR3 loop of a camelid species heavy-chain antibody, wherein said CDR3 loop protrudes from the antigen-binding site and recognizes and interacts with the active site residues of a biologically active target molecule, wherein said biologically active target molecule is a bacterial specific protein, for use in therapy, provided said molecule is not a V_{HH} of an Ig devoid of light chain consisting in the sequence of αTT1 or αTT2 as defined in claim 1.

6. Use of a molecule which is a V_{HH} domain of a camelid heavy chain comprising a CDR3 loop of a camelid species heavy-chain antibody, wherein said CDR3 loop protudes from the antigen-binding site and recognizes and interacts with the active site residues of a biologically active target molecule for the manufacture of a drug for the treatment of infection caused by Salmonella or influenza virus.

7. A molecule as defined in claim 1, for use in the inhibition of the activity of an enzyme.

8. A molecule according to claim 7, wherein said CDR3 loop interacts with the catalytic residues of said enzyme.

9. A molecule which is a V_{HH} domain of a camelid heavy chain antibody comprising a CDR3 loop of a camelid species heavy-chain antibody, wherein said CDR3 loop protrudes from the antigen-binding site and recognizes and interacts with the active site residues of a biologically active target molecule which is an enzyme chosen from the group consisting of HIV protease, HIV reverse transcriptase, SIV protease, alkaline protease from *Pseudomonas aeruginosa,* factor Xa, an RNase, angiogenin, a sialidase, an amylase, a beta-glucanase, and carbonic anhydrase, for use in the inhibition of the activity of said enzyme.

10. A molecule which is a V_{HH} domain of a camelid heavy chain antibody comprising a CDR3 loop of a camelid species heavy-chain antibody, wherein said CDR3 loop protrudes from the antigen-binding site and recognizes and interacts with the active site residues of a biologically active target molecule, wherein said biologically active target molecule is a protease, provided said molecule is not a V_{HH} of an Ig devoid of light chain consisting in the sequence of αTT1 or αTT2 as defined in claim 1, for use in the inhibition of the activity of said protease.

11. A molecule which is a V_{HH} domain of a camelid heavy chain antibody comprising a CDR3 loop of a camelid species heavy-chain antibody, wherein said CDR3 loop protrudes from the antigen-binding site and recognizes and interacts with the active site residues of a biologically active target molecule, wherein said biologically active target molecule is a lysozyme , provided said molecule is not a V_{HH} of an Ig devoid of light chain consisting in the sequence αLYS2 or αLYS3 as defined in claim 1, for use in the inhibition of the activity of said lysozyme.

12. A molecule which is a V_{HH} domain of a camelid heavy chain antibody comprising a CDR3 loop of a camelid species heavy-chain antibody, wherein said CDR3 loop protrudes from the antigen-binding site and recognizes and interacts with the active site residues of a biologically active target molecule wherein said biologically active target molecule is a toxic substance, for use in the inhibition of the deleterious effect of toxic substances by interacting with amino acid residues that are part of the toxic site of said toxic substance, provided said molecule is not a V_{HH} of an Ig devoid of light chain consisting in the sequence of αTT1 or αTT2 as defined in claim 1.

13. A molecule which is a V_{HH} domain of a camelid heavy chain antibody comprising a CDR3 loop of a camelid species heavy-chain antibody, wherein said CDR3 loop protrudes from the antigen-binding site and recognizes and interacts with the active site residues of a biologically active target molecule, wherein said biologically active target molecule is a toxic substance chosen from the group consisting of Toxic Shock Syndrome Toxin 1 from *Staphylococcus aureus,* a staphylococcal enterotoxin B protein, a cholera toxin, a snake venom, adamalysin II from rattlesnake, Cardiotoxin CTX IIb from *Naja Mosambica,* Cardiotoxin CTX V from Taiwan Cobra, Dendrotoxin from Black mamba, Flavoridin Neurotoxin-I and -II from Asian Cobra, low weight metalloproteinase Ht-c and Ht-d from *Crotalus atrox,* a honey bee venom, apamin, tertiapin, and a spider toxin, for use in the inhibition of the deleterious effect of toxic substances by interacting with amino acid residues that are part of the toxic site of said toxic substance.

14. A molecule which is a V_{HH} domain of a camelid heavy chain antibody comprising a CDR3 loop of a camelid species heavy-chain antibody, wherein said CDR3 loop protrudes from the antigen-binding site and recognizes and interacts with the active site residues of a biologically active target molecule, wherein said biologically active target molecule is a bacterial toxin, for use in the inhibition of the deleterious effect of said bacterial toxin by interacting with amino acid residues that are part of the toxic site of said bacterial toxin, provided said molecule is not a V_{HH} of an Ig devoid of light chain consisting in the sequence of αTT1 or αTT2 as defined in claim 1.

15. A molecule according to claim 14, wherein said bacterial toxin is the tetanus toxin.

16. Use of a molecule which is a V_{HH} domain of a camelid heavy chain comprising a CDR3 loop of a camelid species heavy-chain antibody, wherein said CDR3 loop protudes from the antigen-binding site and recognizes and interacts with the active site residues of a biologically active target molecule for the manufacture of a drug for treatment of diseases of parasitic origin.

17. A molecule which is a V_{HH} domain of a camelid heavy chain antibody comprising a CDR3 loop of a camelid species heavy-chain antibody, wherein said CDR3 loop protudes from the antigen-binding site ans recognizes and interacts with the active site residues of a biologically active target molecule, wherein said target molecule is a receptor, for use in therapy.

18. Use of a molecule which is a V_{HH} domain of a camelid heavy chain comprising a CDR3 loop of a camelid species heavy-chain antibody, wherein said CDR3 loop protudes from the antigen-binding site and recognizes and interacts with the active site residues of a biologically active target molecule for the manufacture of diagnostic test kits.

19. A molecule as defined in claim 1 for use in immunization.

## Patentansprüche

1. Molekül zur Verwendung in der Therapie, das eine V_{HH}-Domäne eines Schwere Kette-Antikörpers eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, mit der Maßgabe, dass das Molekül nicht ein V_{HH} ist, das aus einer der folgenden Proteinsequenzen besteht:
i) αTT1: Glu-Val-Gln-Leu-Gln-Ala-Ser-Gly-Gly-Gly-Ser-Val-Gln-Ala-Gly-Gly-Ser-Leu-Arg-Leu-Ser-Cys-Ala-Ala-Ser-Gly-Gly-Gln-Thr-Phe-Asp-Ser-Tyr-Ala-Met-Ala-Trp-Phe-Arg-Gln-Ala-Pro-Gly-Lys-Glu-Cys-Glu-Leu-Val-Ser-Ser-Ile-Ile-Gly-Asp-Asp-Asn-Arg-Asn-Tyr-Ala-Asp-Ser-Val-Lys-Gly-Arg-Phe-Thr-Ile-Ser-Arg-Asg-Asn-Ala-Lys-Asn-Thr-Val-Tyr-Leu-Gln-Met-Asp-Arg-Leu-Asu-Pro-Glu-Asp-Thr-Ala-Val-Tyr-Tyr-Cys-Ala-Gln-Leu-Gly-Ser-Ala-Arg-Ser-Ala-Met-Tyr-Cys-Ala-Gly-Gln-Gly-Thr-Gln-Val-Thr-Val-Ser-Ser
ii) αTT2: Glu-Val-Gln-Leu-Gln-Ala-Ser-Gly-Gly-Gly-Ser-Val-Glu-Ala-Gly-Gly-Ser-Leu-Arg-Leu-Ser-Cys-Thr-Ala-Ala-Asn-Tyr-Ala-Phe-Asp-Ser-Lys-Thr-Val-Gly-Trp-Phe-Arg-Gln-Val-Pro-Gly-Lys-Glu-Arg-Glu-Gly-Val-Ala-Gly-Ile-Ser-Ser-Gly-Gly-Ser-Thr-Thr-Ala-Tyr-Ser-Asp-Ser-Val-Lys-Gly-Arg-Tyr-Thr-Val-Ser-Leu-Glu-Asn-Ala-Lys-Asn-Thr-Val-Tyr-Leu-Leu-Ile-Asp-Asn-Leu-Gln-Pro-Glu-Asp-Thr-Ala-Ile-Tyr-Tyr-Cys-Ala-Gly-Val-Ser-Gly-Trp-Arg-Gly-Arg-Gln-Trp-Leu-Leu-Leu-Ala-Glu-Thr-Tyr-Arg-Phe-Trp-Gly-Gln-Gly-Thr-Gln-Val-Thr-Val-Ser-Ser
iii) αLYS2: Glu-Val-Gln-Leu-Gln-Ala-Ser-Gly-Gly-Gly-Ser-Val-Gln-Ala-Gly-Gln-Ser-Leu-Arg-Leu-Ser-Cys-Ala-Thr-Ser-Gly-Ala-Thr-Ser-Ser-Asn-Ser-Cys-Met-Gly-Trp-Phe-Arg-Gln-Ala-Pro-Gly-Lys-Glu-Arg-Glu-Gly-Val-Ala-Val-Ile-Asp-Thr-Gly-Arg-Gly-Asn-Thr-Ala-Tyr-Ala-Asp-Ser-Val-Gln-Gly-Arg-Leu-Thr-Ile-Ser-Leu-Asp-Asn-Ala-Lys-Asn-Thr-Leu-Tyr-Leu-Gln-Met-Asn-Ser-Leu-Lys-Pro-Glu-Asp-Thr-Ala-Met-Tyr-Tyr-Cys-Ala-Ala-Asp-Thr-Ser-Thr-Trp-Tyr-Arg-Gly-Tyr-Cys-Gly-Thr-Asn-Pro-Asn-Tyr-Phe-Ser-Tyr-Trp-Gly-Gln-Gly-Tbr-Gln-Val-Thr-Val-Ser-Ser, und
iv) αLYS3: Asp-Val-Gln-Leu-Gln-Ala-Ser-Gly-Gly-Gly-Ser-Val-Gln-Ala-Gly-Gly-Ser-Leu-Arg-Leu-Ser-Cys-Ala-Ala-Ser-Gly-Tyr-Tlu-Ile-Gly-Pro- Tyr-Cys-Met-Gly-Trp-Phe-Arg-Gln-Ala-Pro-Gly-Lys-Glu-Arg-Glu-Gly-Val-Ala-Ala-Ile-Asn-Met-Gly-Gly-Gly-Ile-Thr-Tyr-Tyr-Ala-Asp-Ser-Val-Lys-Gly-Arg-Phe-Thr-Ile-Ser-Gln-Asp-Asn-Ala-Lys-Asn-Thr-Val-Tyr-Leu-Leu-Met-Asn-Ser-Leu-Glu-Pro-Glu-Asp-Thr-Ala-Ile-Tyr-Tyr-Cys- Ala-Ala-Asp-Ser-Thr-Ile-Tyr-Ala-Ser-Tyr-Tyr-Glu-Cys-Gly-His-Gly-Leu-Ser-Thr-Gly-Gly-Tyr-Gly-Tyr-Asp-Ser-Trp-Gly-Gln-Gly-Thr-Gln-Val-Thr-Val-Ser-Ser.

2. Molekül wie in Anspruch 1 definiert zur Verwendung in der Neutralisierung der biologischen Funktion des biologisch aktiven Zielmoleküls.

3. Verwendung eines Moleküls, das eine V_{HH}-Domäne einer schweren Kette eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, für die Herstellung eines Medikaments zur Behandlung pankreatischer Störungen.

4. Molekül zur Verwendung in der Therapie, das eine V_{HH}-Domäne eines Schwere Kette-Antikörpers eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, wobei das biologisch aktive Zielmolekül ein virusspezifisches Protein ist.

5. Molekül zur Verwendung in der Therapie, das eine V_{HH}-Domäne eines Schwere Kette-Antikörpers eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, wobei das biologisch aktive Zielmolekül ein spezifisches bakterielles Protein ist, mit der Maßgabe, dass das Molekül nicht ein V_{HH} eines Ig ist, das ohne leichte Kette ist, bestehend aus der Sequenz von αTT1 oder αTT2 wie in Anspruch 1 definiert.

6. Verwendung eines Moleküls, das eine V_{HH}-Domäne einer schweren Kette eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, für die Herstellung eines Medikaments zur Behandlung einer durch Salmonella oder Influenza-Virus verursachten Infektion.

7. Molekül wie in Anspruch 1 definiert zur Verwendung in der Hemmung der Aktivität eines Enzyms.

8. Molekül nach Anspruch 7, wobei die CDR3-Schleife mit den katalytischen Resten des Enzyms wechselwirkt.

9. Molekül, das eine V_{HH}-Domäne eines Schwere Kette-Antikörpers eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, wobei das biologisch aktive Zielmolekül ein Enzym ist, das ausgewählt ist aus der Gruppe bestehend aus HIV-Protease, HIV-reverse Transkriptase, SIV-Protease, alkalische Protease von *Pseudomonas aeruginosa,* Faktor Xa, einer RNase, Angiogenin, einer Sialidase, einer Amylase, einer beta-Glucanase und Carboanhydrase, zur Verwendung in der Hemmung der Aktivität des Enzyms.

10. Molekül, das eine V_{HH}-Domäne eines Schwere Kette-Antikörpers eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, wobei das biologisch aktive Zielmolekül eine Protease ist, zur Verwendung in der Hemmung der Aktivität der Protease, mit der Maßgabe, dass das Molekül nicht ein V_{HH} eines Ig ist, das ohne leichte Kette ist, bestehend aus der Sequenz von αTT1 oder αTT2 wie in Anspruch 1 definiert.

11. Molekül, das eine V_{HH}-Domäne eines Schwere-Kette-Antikörpers eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, wobei das biologisch aktive Zielmolekül ein Lysozym ist, zur Verwendung in der Hemmung der Aktivität des Lysozyms, mit der Maßgabe, dass das Molekül nicht ein V_{HH} eines Ig ist, das ohne leichte Kette ist, bestehend aus der Sequenz von αLYS2 oder αLYS3 wie in Anspruch 1 definiert.

12. Molekül, das eine V_{HH}-Domäne eines Schwere Kette-Antikörpers eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, wobei das biologisch aktive Zielmolekül eine toxische Substanz ist, zur Verwendung in der Hemmung des schädlichen Effekts toxischer Substanzen durch Wechselwirken mit Aminosäureresten, die Teil der toxischen Stelle der toxischen Substanz sind, mit der Maßgabe, dass das Molekül nicht ein V_{HH} eines lg ist, das ohne leichte Kette ist, bestehend aus der Sequenz von αTT1 oder αTT2 wie in Anspruch 1 definiert.

13. Molekül, das eine V_{HH}-Domäne eines Schwere Kette-Antikörpers eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, wobei das biologisch aktive Zielmolekül eine toxische Substanz ist, ausgewählt aus der Gruppe bestehend aus Toxisches-Schock-Syndrom-Toxin 1 aus *Staphylococcus aureus,* einem Enterotoxin B-Protein aus Staphylokokken, einem Choleratoxin, einem Schlangengift, Adamalysin II der Klapperschlange, Cardiotoxin CTX Ilb aus *Naja Mosambica,* Cardiotoxin CTX V der Taiwan-Kobra, Dendrotoxin der Schwarzen Mamba, Flavoridin-Neurotoxin-I und -II der Asiatischen Kobra, Metalloproteinase niedrigen Gewichts Ht-c und Ht-d aus *Crotalus atrox,* einem Honigbienengift, Apamin, Tertiapin und einem Spinnengift, zur Verwendung in der Hemmung des schädlichen Effekts toxischer Substanzen durch Wechselwirken mit Aminosäureresten, die Teil der toxischen Stelle der toxischen Substanz sind.

14. Molekül, das eine V_{HH}-Domäne eines Schwere Kette-Antikörpers eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, wobei das biologisch aktive Zielmolekül ein bakterielles Toxin ist, zur Verwendung in der Hemmung des schädlichen Effekts des bakteriellen Toxins durch Wechselwirken mit Aminosäureresten, die Teil der toxischen Stelle des bakteriellen Toxins sind, mit der Maßgabe, dass das Molekül nicht ein V_{HH} eines lg ist, das ohne leichte Kette ist, bestehend aus der Sequenz von αTT1 oder αTT2 wie in Anspruch 1 definiert.

15. Molekül nach Anspruch 14, wobei das bakterielle Toxin Tetanustoxin ist.

16. Verwendung eines Moleküls, das eine V_{HH}-Domäne einer schweren Kette eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, für die Herstellung eines Medikaments für die Behandlung von Krankheiten parasitischen Ursprungs.

17. Molekül zur Verwendung in der Therapie, das eine V_{HH}-Domäne eines Schwere Kette-Antikörpers eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, wobei das Zielmolekül ein Rezeptor ist.

18. Verwendung eines Moleküls, das eine V_{HH}-Domäne einer schweren Kette eines Cameliden ist, umfassend eine CDR3-Schleife eines Schwere Kette-Antikörpers einer Camelidenart, wobei die CDR3-Schleife aus der Antigen-Bindungsstelle herausragt und die Reste des aktiven Zentrums eines biologisch aktiven Zielmoleküls erkennt und damit wechselwirkt, zur Herstellung diagnostischer Testkits.

19. Molekül wie in Anspruch 1 definiert zur Verwendung in der Immunisierung.

## Revendications

1. Molécule qui est un domaine V_{HH} d'un anticorps à chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active,
à condition que ladite molécule ne soit pas un V_{HH} consistant en l'une des séquences protéïques suivantes :
i) αTT1 : Glu-Val-Gln-Leu-Gln-Ala-Ser-Gly-Gly-Gly-SerVal-Gln-Ala-Gly-Gly-Ser-Leu-Arg-Leu-Ser-Cys-Ala-Ala-Ser-Gly-Gly-Gln-Thr-Phe-Asp-Ser-Tyr-Ala-Met-Ala-Trp-Phe-Arg-Gln-Ala-Pro-Gly-Lys-Glu-Cys-Glu-Leu-Val-Ser-Ser-Ile-Ile-Gly-Asp-Asp-Asn-Arg-Asn-Tyr-Ala-Asp-SerVal-Lys-Gly-Arg-Phe-Thr-Ile-Ser-Arg-Asp-Asn-Ala-Lys-Asn-Thr-Val-Tyr-Leu-Gln-Met-Asp-Arg-Leu-Asn-Pro-Glu-Asp-Thr-Ala-Val-Tyr-Tyr-Cys-Ala-Gln-Leu-Gly-Ser-Ala-Arg-Ser-Ala-Met-Tyr-Cys-Ala-Gly-Gln-Gly-Thr-Gln-Val-Thr-Val-Ser-Ser,
ii) αTT2 : Glu-Val-Gln-Leu-Gln-Ala-Ser-Gly-Gly-Gly-SerVal-Gln-Ala-Gly-Gly-Ser-Leu-Arg-Leu-Ser-Cys-Thr-Ala-Ala-Asn-Tyr-Ala-Phe-Asp-Ser-Lys-Thr-Val-Gly-Trp-Phe-Arg-Gln-Val-Pro-Gly-Lys-Glu-Arg-Glu-Gly-Val-Ala-Gly-Ile-Ser-Ser-Gly-Gly-Ser-Thr-Thr-Ala-Tyr-Ser-Asp-SerVal-Lys-Gly-Arg-Tyr-Thr-Val-Ser-Leu-Glu-Asn-Ala-Lys-Asn-Thr-Val-Tyr-Leu-Leu-Ile-Asp-Asn-Leu-Gln-Pro-Glu-Asp-Thr-Ala-Ile-Tyr-Tyr-Cys-Ala-Gly-Val-Ser-Gly-Trp-Arg-Gly-Arg-Gln-Trp-Leu-Leu-Leu-Ala-Glu-Thr-Tyr-Arg-Phe-Trp-Gly-Gln-Gly-Thr-Gln-Val-Thr-Val-Ser-Ser,
iii) αLYS2 : Glu-Val-Gln-Leu-Gln-Ala-Ser-Gly-Gly-Gly-Ser-Val-Gln-Ala-Gly-Gln-Ser-Leu-Arg-Leu-Ser-Cys-Ala-Thr-Ser-Gly-Ala-Thr-Ser-Ser-Asn-Ser-Cys-Met-Gly-Trp-Phe-Arg-Gln-Ala-Pro-Gly-Lys-Glu-Arg-Glu-Gly-Val-Ala-Val-Ile-Asp-Thr-Gly-Arg-Gly-Asn-Thr-Ala-Tyr-Ala-Asp-Ser-Val-Gln-Gly-Arg-Leu-Thr-Ile-Ser-Leu-Asp-Asn-Ala-Lys-Asn-Thr-Leu-Tyr-Leu-Gln-Met-Asn-Ser-Leu-Lys-Pro-Glu-Asp-Thr-Ala-Met-Tyr-Tyr-Cys-Ala-Ala-Asp-Thr-Ser-Thr-Trp-Tyr-Arg-Gly-Tyr-Cys-Gly-Thr-Asn-Pro-Asn-Tyr-Phe-Ser-Tyr-Trp-Gly-Gln-Gly-Thr-Gln-Val-Thr-Val-Ser-Ser, et
iv) αLYS3 : Asp-Val-Gln-Leu-Gln-Ala-Ser-Gly-Gly-Gly-SerVal-Gln-Ala-Gly-Gly-Ser-Leu-Arg-Leu-Ser-Cys-Ala-Ala-Ser-Gly-Tyr-Thr-Ile-Gly-Pro-Tyr-Cys-Met-Gly-Trp-Phe-Arg-Gln-Ala-Pro-Gly-Lys-Glu-Arg-Glu-Gly-Val-Ala-Ala-Ile-Asn-Met-Gly-Gly-Gly-Ile-Thr-Tyr-Tyr-Ala-Asp-Ser-Val-Lys-Gly-Arg-Phe-Thr-Ile-Ser-Gln-Asp-Asn-Ala-Lys-Asn-Thr-Val-Tyr-Leu-Leu-Met-Asn-Ser-Leu-Glu-Pro-Glu-Asp-Thr-Ala-Ile-Tyr-Tyr-Cys-Ala-Ala-Asp-Ser-Thr-Ile-Tyr-Ala-Ser-Tyr-Tyr-Glu-Cys-Gly-His-Gly-Leu-Ser-Thr-Gly-Gly-Tyr-Gly-Tyr-Asp-Ser-Trp-Gly-Gln-Gly-Thr-Gln-Val-Thr-Val-Ser-Ser, pour utilisation en thérapie.

2. Molécule selon la revendication 1, pour utilisation dans la neutralisation de la fonction biologique de ladite molécule cible biologiquement active.

3. Utilisation d'une molécule qui est un domaine V_{HH} d'une chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active, pour la fabrication d'un médicament destiné au traitement de troubles du pancréas.

4. Molécule qui est un domaine V_{HH} d'un anticorps à chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active, dans laquelle ladite molécule cible biologiquement active est une protéine spécifiquement virale, pour utilisation en thérapie.

5. Molécule qui est un domaine V_{HH} d'un anticorps à chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active, dans laquelle ladite molécule cible biologiquement active est une protéine spécifiquement bactérienne, pour utilisation en thérapie, à condition que ladite molécule ne soit pas un V_{HH} d'une Ig dépourvue de chaîne légère consistant en la séquence de αTT1 ou αTT2 telles que définies dans la revendication 1.

6. Utilisation d'une molécule qui est un domaine V_{HH} d'une chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active, pour la fabrication d'un médicament destiné au traitement d'une infection causée par *Salmonella* ou le virus *influenza.*

7. Molécule selon la revendication 1, pour utilisation dans l'inhibition de l'activité d'une enzyme.

8. Molécule selon la revendication 7, dans laquelle ladite boucle CDR3 interagit avec les résidus catalytiques de ladite enzyme.

9. Molécule qui est un domaine V_{HH} d'un anticorps à chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active qui est une enzyme choisie dans le groupe constitué par la protéase du VIH, la transcriptase inverse du VIH, la protéase du VIS, la protéase alcaline de *Pseudomonas aeruginosa,* le facteur Xa, une RNase, l'angiogénine, une sialidase, une amylase, une bêta-glucanase et une anhydrase carbonique, pour utilisation dans l'inhibition de l'activité de ladite enzyme.

10. Molécule qui est un domaine V_{HH} d'un anticorps à chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active, dans laquelle ladite molécule cible biologiquement active est une protéase, à condition que ladite molécule ne soit pas un V_{HH} d'une Ig dépourvue de chaîne légère consistant en la séquence de αTT1 ou αTT2 telles que définies dans la revendication 1, pour utilisation dans l'inhibition de l'activité de ladite protéase.

11. Molécule qui est un domaine V_{HH} d'un anticorps à chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active, dans laquelle ladite molécule cible biologiquement active est un lysozyme, à condition que ladite molécule ne soit pas un V_{HH} d'une Ig dépourvue de chaîne légère consistant en la séquence de αLYS2 ou αLYS3 telles que définies dans la revendication 1, pour utilisation dans l'inhibition de l'activité dudit lysozyme.

12. Molécule qui est un domaine V_{HH} d'un anticorps à chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active, dans laquelle ladite molécule cible biologiquement active est une substance toxique, pour utilisation dans l'inhibition de l'effet délétère de substances toxiques par interaction avec des résidus d'acides aminés qui font partie du site toxique de ladite substance toxique, à condition que ladite molécule ne soit pas un V_{HH} d'une Ig dépourvue de chaîne légère consistant en la séquence de αTT1 ou αTT2 telles que définies dans la revendication 1.

13. Molécule qui est un domaine V_{HH} d'un anticorps à chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active, dans laquelle ladite molécule cible biologiquement active est une substance toxique choisie dans le groupe constitué par la toxine 1 du syndrome de choc toxique de *Staphylococcus aureus,* une protéine B d'entérotoxine staphylococcique, une toxine cholérique, un venin de serpent, l'adamalysine II du serpent à sonnette, la cardiotoxine CTX IIb de *Naja Mossambica,* la cardiotoxine CTX V du cobra de Taiwan, la dendrotoxine du mamba noir, la neurotoxine flavoridine I et II du cobra asiatique, la métalloprotéase de faible poids Ht-c et Ht-d de *Crotalus atrox,* un venin d'abeille, l'apamine, la tertiapine et une toxine d'araignée, pour utilisation dans l'inhibition de l'effet délétère des substances toxiques par interaction avec des résidus d'acides aminés qui font partie du site toxique de ladite substance toxique.

14. Molécule qui est un domaine V_{HH} d'un anticorps à chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active, dans laquelle ladite molécule cible biologiquement active est une toxine bactérienne, pour utilisation dans l'inhibition de l'effet délétère de ladite toxine bactérienne par interaction avec des résidus d'acides aminés qui font partie du site toxique de ladite toxine bactérienne, à condition que ladite molécule ne soit pas un V_{HH} d'une Ig dépourvue de chaîne légère consistant en la séquence de αTT1 ou αTT2 telles que définies dans la revendication 1.

15. Molécule selon la revendication 14, dans laquelle ladite toxine bactérienne est la toxine tétanique.

16. Utilisation d'une molécule qui est un domaine V_{HH} d'une chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active, pour la fabrication d'un médicament destiné au traitement de maladies d'origine parasitaire.

17. Molécule qui est un domaine V_{HH} d'un anticorps à chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active, dans laquelle ladite molécule cible est un récepteur, pour utilisation en thérapie.

18. Utilisation d'une molécule qui est un domaine V_{HH} d'une chaîne lourde de camélidé comprenant une boucle CDR3 d'un anticorps à chaîne lourde d'une espèce de camélidé, dans laquelle ladite boucle CDR3 dépasse du site de liaison à l'antigène et reconnaît et interagit avec les résidus du site actif d'une molécule cible biologiquement active, pour la fabrication de kits de test diagnostic.

19. Molécule selon la revendication 1 pour utilisation en immunisation.
